# EUROPEAN PATENT SPECIFICATION

(11) **EP 1 713 501 B1**
(45) Date of publication and mention of the grant of the patent: **16.01.2008**
(21) Application number: 04803345.0
(22) Date of filing: 29.11.2004
(51) Int. Cl.: A61K 39/00

(54) **TREATMENT OF NEURODEGENERATIVE DISEASES BY THE USE OF GPR49**
BEHANDLUNG VON NEURODEGENERATIVE KRANKHEITEN MIT GPR49
TRAITEMENT DE MALADIES NEURODEGENERATIVES AU MOYEN DE GPR49

(30) Priority: 29.01.2004 EP 04001895; 29.01.2004 EP 04001894; 26.03.2004 EP 04007447; 07.05.2004 WO PCT/EP2004/004891; 07.05.2004 WO PCT/EP2004/004889
(43) Date of publication of application: 25.10.2006
(73) Proprietor: CELLZOME AG, 69117 Heidelberg (DE)
(72) Inventor: HOPF, Carsten, 68165 Mannheim (DE); DREWES, Gerard, 69121 Heidelberg (DE); RUFFNER, Heinz, 69245 Bammental (DE)
(74) Representative: Huhn, Michael
(86) International application number: PCT/EP2004/013539
(87) International publication number: WO 2005/074980

(56) References cited:
- WO-A-00/43039

## Description

The present invention relates to protein complexes of the APP-processing pathway comprising the GPR49 protein as well as to the use of inhibitors of these complexes as well as of GPR49 in the treatment of neurogenerative diseases.

Alzheimer's disease is a chronic condition that affects millions of individuals worldwide.

The brains of sufferers of Alzheimer's disease show a characteristic pathology of prominent neuropathologic lesions, such as the initially intracellular neurofibrillary tangles (NFTs), and the extracellular amyloid-rich senile plaques. These lesions are associated with massive loss of populations of CNS neurons and their progression accompanies the clinical dementia associated, with AD. The major component of amyloid plaques are the amyloid beta (A-beta, Abeta or AB) peptides of various lengths. A variant thereof, which is the AB1-42-peptide (Abeta-42), is the major causative agent for amyloid formation. Another variant is the AB1-40-peptide (Abeta-40). Amyloid beta is the proteolytic product of a precursor protein, beta amyloid precursor protein (beta-APP or APP). APP is a type-I trans-membrane protein which is sequentially cleaved by several different membrane-associated proteases. The first cleavage of APP occurs by one of two proteases, alpha-secretase or beta-secretase. Alpha-secretase is a metalloprotease whose activity is most likely to be provided by one or a combination of the proteins ADAM-10 and ADAM-17. Cleavage by alpha-secretase precludes formation of amyloid peptides and is thus referred to as non-amyloidogenic. In contrast, cleavage of APP by beta-secretase is a prerequisite for subsequent formation of amyloid peptides. This secretase, also called BACE1 (beta-site APP-cleaving enzyme), is a type-I transmembrane protein containing an aspartyl protease activity (described in detail below).

The beta-secretase (BACE) activity cleaves APP in the ectodomain, resulting in shedding of secreted, soluble APPb, and in a 99-residue C-terminal transmembrane fragment (APP-C99). Vassar et al. (Science 286, 735-741) cloned a transmembrane aspartic protease that had the characteristics of the postulated beta-secretase of APP, which they termed BACE1. Brain and primary cortical cultures from BACE1 knockout mice showed no detectable beta-secretase activity, and primary cortical cultures from BACE knockout mice produced much less amyloid-beta from APP. This suggests that BACE1, rather than its paralogue BACE2, is the main beta-secretase for APP. BACE1 is a protein of 501 amino acids (aa) containing a 21-aa signal peptide followed by a prosequence domain spanning aa 22 to 45. There are alternatively spliced forms, BACE-I-457 and BACE-I-476. The extracellular domain of the mature protein is followed by one predicted transmembrane domain and a short cytosolic C-terminal tail of 24 aa. BACE1 is predicted to be a type 1 transmembrane protein with the active site on the extracellular side of the membrane, where beta-secretase cleaves APP and possible other yet unidentified substrates. Although BACE1 is clearly a key enzyme required for the processing of APP into A-beta, recent evidence suggests additional potential substrates and functions of BACE1 (J. Biol. Chem. 279, 10542-10550). To date, no BACE1 interacting proteins with regulatory or modulatory functions have been described.

The APP fragment generated by BACE1 cleavage, APP-C99, is a substrate for the gamma-secretase activity, which cleaves APP-C99 within the plane of the membrane into an A-beta peptide (such as the amyloidogenic AB1-42 peptide), and into a C-terminal fragment termed APP intracellular domain (AICD) (Annu Rev Cell Dev Biol 19, 25-51). The gamma-secretase activity resides within a multiprotein complex with at least four distinct subunits. The first subunit to be discovered was presenilin (Proc Natl Acad Sci USA 94, 8208-13). Other known protein components of the gamma-secretase complex are Pen-2, Nicastrin and Aph-1a (Aph1a).

Despite recent progress in delineating molecular events underlying the etiology of Alzheimer's disease, no disease-modifying therapies have been developed so far. To this end, the industry has struggled to identify suitable lead compounds for inhibition of BACE1. Moreover, it has been recognized that a growing number of alternative substrates of gamma-secretase exist, most notably the Notch protein. Consequently, inhibition of gamma-secretase is likely to cause mechanism-based side effects. Current top drugs (e.g. Aricept®/donepezil) attempt to achieve a temporary improvement of cognitive functions by inhibiting acetylcholinesterase, which results in increased levels of the neurotransmitter acetylcholine in the brain. These therapies are not suitable for later stages of the disease, they do not treat the underlying disease pathology, and they do not halt disease progression.

Thus, there is an uninet need for the identification of novel targets allowing novel molecular strategies for the treatment of Alzheimer's disease. In addition, there is a strong need for novel therapeutic compounds modifying the aforementioned molecular processes by targeting said novel targets.

In a first aspect, the invention provides the use of a "GPR49 inhibitor selected from the group consisting of antibodies, antisense oligonucleotides in RNA and ribozymes for the preparation of a pharmaceutical composition for the treatment of neurogenerative diseases.

In the context of the present invention, it has been surprisingly found that the GPR49 (also known as leucine-rich repeat-containing G protein-coupled receptor 5 (LGR5); FEX HG38, GPR67) forms part of different protein complexes which are involved in the aberrant processing of APP in Alzheimer's disease by gamma-secretase. Especially, it has been found that GPR49 is part of the Aphla-complex, of the Fe65L2-complex, of the APP-C99-complex, and of the BACE1-complex. These complexes are named after their respective key protein compound that has been used as the TAP technology entry point (see below).

The identification of GPR49 as a key molecule in these complexes enables the use of molecules interacting with GPR49 for the treatment of neurodegenerative diseases. This is especially shown in the examples where it is demonstrated that siRNA directed against GPR49 results in attenuation of generation and/or secretion of Abeta-42.

In the context of the present invention, a "GPR49 interacting molecule" is a molecule which binds at least temporarily to GPR49 and which preferably modulates and particularly inhibits GPR49 activity.

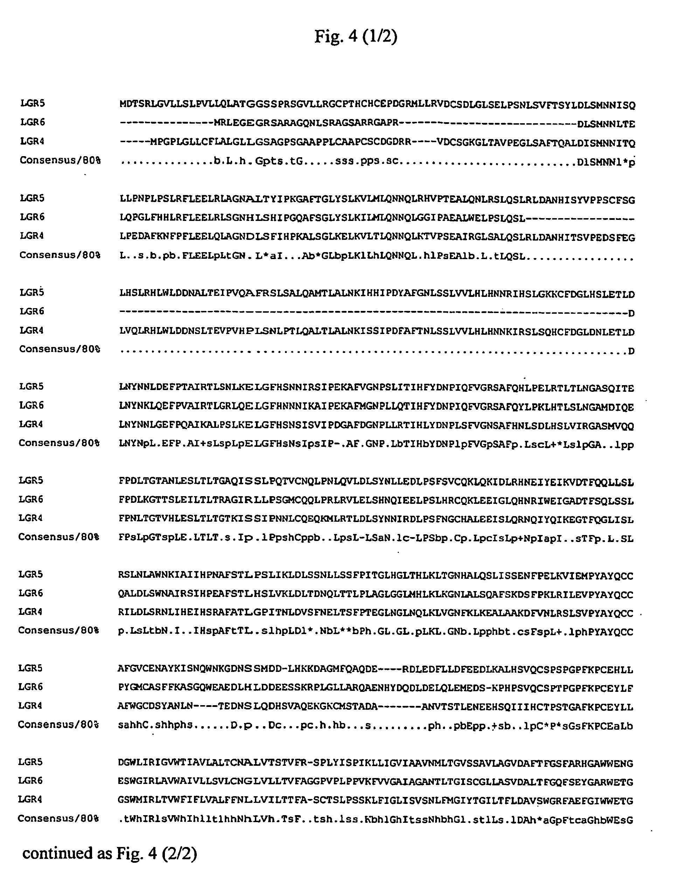

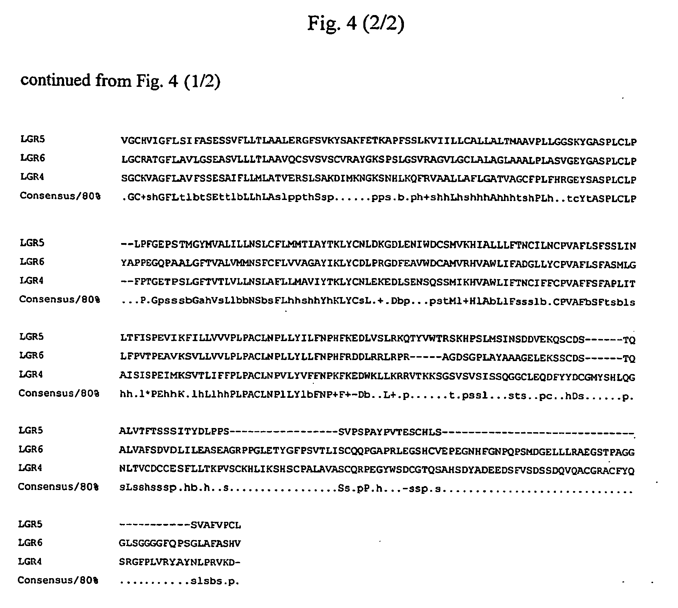

GPR49 is a putative member of the glycoprotein hormone receptor superfamily - unusual G-protein-coupled receptors (GPCRs) that are characterized by a large N-tenninal ectodomain containing leucine-rich repeats that in some cases have been shown to be important for interaction with glycoprotein ligands. Phylogenetic analysis of superfamily members suggests that it is divided into three sub-families - the first featuring receptors for LH, FSH (follicle-stimulating hormone) and TSH (thyroid-stimulating hormone); the second containing relaxin receptors LGR7 and LGR8. GPR49/LGR5 together with LGR4 and LGR6 (Fig. 4) constitutes a sub-family of orphan receptors that display only ~35% sequence identity with the FSH receptor (Hsu SY, Liang SG, Hsueh AJ (1998) Characterization of two LGR genes homologous to gonadotropin and thyrotropin receptors with extracellular leucine-rich repeats and a G protein-coupled, seven-transmembrane region. Mol Endocrinol. 12(12):1830-45; McDonald T, Wang R, Bailey W, Xie G, Chen F, Caskey CT, Liu Q (1998) Identification and cloning of an orphan G protein-coupled receptor of the glycoprotein hormone receptor subfamily. Biochem Biophys Res Commun. 247(2):266-70).

GPR49 is prominently expressed in skeletal muscle, placenta and spinal cord and at lower levels in colon, adrenal and various subregions of brain (Hsu SY, et al. (1998), supra). In the adult mouse brain GPR49 transcripts are mostly restricted to the olfactory bulb, where they are found in neuronal cell layers (Hermey G, Methner A, Schaller HC, Hermans-Borgmeyer I (1999) Identification of a novel seven-transmembrane receptor with homology to glycoprotein receptors and its expression in the adult and developing mouse. Biochem Biophys Res Commun. 254(1):273-9). The function of GPR49 in the adult is not well characterized. Targeted deletion of the GPR49 gene in mice results in neonatal lethality and is associated with ankyloglossia and gastrointestinal distension (Morita H, Mazerbourg S, Bouley DM, Luo CW, Kawamura K, Kuwabara Y, Baribault H, Tian H, Hsueh AJ (2004) Neonatal lethality of LGR5 null mice is associated with ankyloglossia and gastrointestinal distension. Mol Cell Biol. 24(22):9736-43).

GPR49 has recently been identified as a gene over-expressed in human hepatocellular carcinomas with beta-catenin mutations (Yamamoto Y, Sakamoto M, Fujii G, Tsuiji H, Kenetaka K, Asaka M, Hirohashi S (2003) Overexpression of orphan G-protein-coupled receptor, Gpr49, in human hepatocellular carcinomas with beta-catenin mutations. Hepatology. 37(3):528-33).

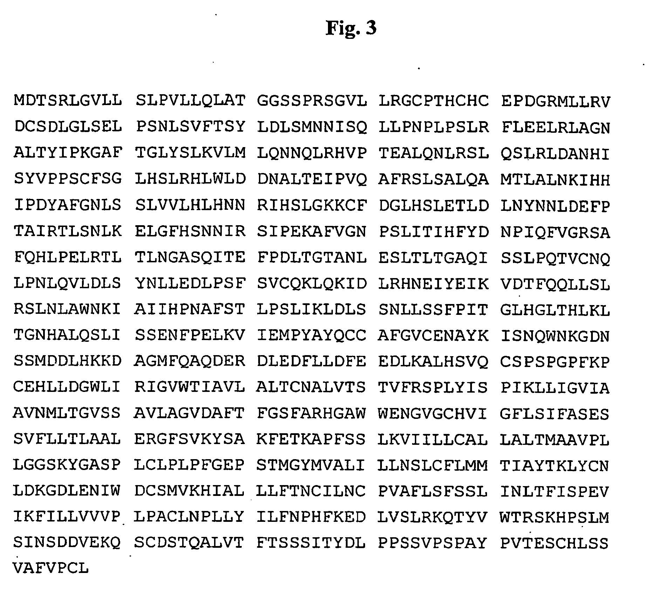

According to the present invention, the expression "GPR49" does not only mean the protein as shown in Fig. 3, but also a functionally active derivative thereof, or a functionally active fragment thereof, or a homologue thereof, or a variant encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

The same applies also to all other proteins named in the present invention. Therefore, a name of given protein or nucleic acid does not only refer to the protein or nucleic acid as depicted in the sequence listing, but also to its functionally active derivative, or to a functionally active fragment thereof, or a homologue thereof, or a variant encoded by a nucleic acid that hybridizes to the nucleic acid encoding said protein under low stringency conditions, preferably under the conditions as mentioned above.

The term "functionally active" as used herein refers to a polypeptide, namely a fragment or derivative, having structural, regulatory, or biochemical functions of the protein according to the embodiment of which this polypeptide, namely fragment or derivative, is related to.

According to the present invention, the term "activity" as used herein, refers to the function of a molecule in its broadest sense. It generally includes, but is not limited to, biological, biochemical, physical or chemical functions of the molecule. It includes for example the enzymatic activity, the ability to interact with other molecules and ability to activate, facilitate, stabilize, inhibit, suppress or destabilize the function of other molecules, stability, ability to localize to certain subcellular locations. Where applicable, said term also relates to the function of a protein complex in its broadest sense.

According to the present invention, the terms "derivatives" or "analogs of component proteins" or "variants" as used herein preferably include, but are not limited, to molecules comprising regions that are substantially homologous to the component proteins, in various embodiments, by at least 30%, 40%, 50%, 60%, 70%, 80%, 90%, 95% or 99% identity over an amino acid sequence of identical size or when compared to an aligned sequence in which the alignment is done by a computer homology program known in the art, or whose encoding nucleic acid is capable of hybridizing to a sequence encoding the component protein under stringent, moderately stringent, or nonstringent conditions. It means a protein which is the outcome of a modification of the naturally occurring protein, by amino acid substitutions, deletions and additions, respectively, which derivatives still exhibit the biological function of the naturally occurring protein although not necessarily to the same degree. The biological function of such proteins can e.g. be examined by suitable available in vitro assays as provided in the invention.

The term "fragment" as used herein refers to a polypeptide of at least 10, 20, 30, 40 or 50 amino acids of the component protein according to the embodiment. In specific embodiments, such fragments are not larger than 35, 100 or 200 amino acids.

The term "gene" as used herein refers to a nucleic acid comprising an open reading frame encoding a polypeptide of, if not stated otherwise, the present invention, including both exon and optionally intron sequences.

The terms " homologue" or "homologous gene products" as used herein mean a protein in another species, preferably mammals, which performs the same biological function as the a protein component of the complex further described herein. Such homologues are also termed "orthologous gene products". The algorithm for the detection of orthologue gene pairs from humans and mammalians or other species uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given, e.g., in Nature, 2001, 409:860-921. The homologues of the proteins according to the invention can either be isolated based on the sequence homology of the genes encoding the proteins provided herein to the genes of other species by cloning the respective gene applying conventional technology and expressing the protein from such gene, or by isolating proteins of the other species by isolating the analogous complex according to the methods provided herein or to other suitable methods commonly known in the art.

According to the present invention the term "GPR49-inhibitor" refers to a GPR 49-interacting molecule being a biochemical or chemical compound which inhibits or reduces the activity of GPR49. This can e.g. occur via suppression of the expression of the corresponding gene. The expression of the gene can be measured by RT-PCR or Western blot analysis. Furthermore, this can occur via inhibition of the activity, e.g. by binding to GPR49.

Examples of such GPR49-inhibitors are binding proteins or binding peptides directed against GPR49, in particular against the active site of GPR49, and nucleic acids directed against the GPR49 gene.

The term "nucleic acids against GPR49" refers to double-stranded or single stranded DNA or RNA, or a modification or derivative thereof which, for example, inhibit the expression of the GPR49 gene or the activity of GPR49 and includes, without limitation, antisense nucleic acids, aptamers, siRNAs (small interfering RNAs) and ribozymes.

The inhibitor may be selected from the group consisting of antibodies, antisense oligonucleotides, siRNA, low molecular weight molecules (LMWs), binding peptides, aptamers, ribozymes and peptidomimetics.

These nucleic acids can be directly administered to a cell, or which can be produced intracellularly by transcription of exogenous, introduced sequences.

An "antisense" nucleic acid as used herein refers to a nucleic acid capable of hybridizing to a sequence-specific portion of a component protein RNA (preferably mRNA) by virtue of some sequence complementarity. The antisense nucleic acid may be complementary to a coding and/or noncoding region of a component protein mRNA. Such antisense nucleic acids that inhibit complex formation or activity have utility as therapeutics, and can be used in the treatment or prevention of disorders as described herein.

The antisense nucleic acids are of at least six nucleotides and are preferably oligonucleotides, ranging from 6 to about 200 nucleotides. In specific aspects, the oligonucleotide is at least 10 nucleotides, at least 15 nucleotides, at least 100 nucleotides, or at least 200 nucleotides.

The nucleic acids, e.g. the antisense nucleic acids or siRNAs, can be synthesized chemically, e.g. in accordance with the phosphotriester method (see, for example, Uhlmann, E. & Peyman, A. (1990) Chemical Reviews, 90, 543-584). Aptamers are nucleic acids which bind with high affinity to a polypeptide, here GPR49. Aptamers can be isolated by selection methods such as SELEX (see e.g. Jayasena (1999) Clin. Chem., 45, 1628-50; Klug and Famulok (1994) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1996) Nat. Biotechnol., 14, 1116-9; Klussmann et al. (1996) Nat. Biotechnol., 14, 1112-5). Forms which have been isolated in this way enjoy the advantage that they are not degraded by naturally occurring ribonucleases and, therefore, possess greater stability.

Nucleic acids may be degraded by endonucleases or exonucleases, in particular by DNases and RNases which can be found in the cell. It is, therefore, advantageous to modify the nucleic acids in order to stabilize them against degradation, thereby ensuring that a high concentration of the nucleic acid is maintained in the cell over a long period of time (Beigelman et al. (1995) Nucleic Acids Res. 23:3989-94; WO 95/11910; WO 98/37240; WO 97/29116). Typically, such a stabilization can be obtained by introducing one or more internucleotide phosphorus groups or by introducing one or more non-phosphorus internucleotides.

Suitable modified intemucleotides are compiled in Uhlmann and Peyman (1990), supra (see also Beigelman et al. (1995) Nucleic Acids Res. 23:3989-94; WO 95/11910; WO 98/37240; WO 97/29116). Modified internucleotide phosphate radicals and/or non-phosphorus bridges in a nucleic acid which can be employed in one of the uses according to the invention contain, for example, methyl phosphonate, phosphorothioate, phosphoramidate, phosphorodithioate and/or phosphate esters, whereas non-phosphorus internucleotide analogues contain, for example, siloxane bridges, carbonate bridges, carboxymethyl esters, acetamidate bridges and/or thioether bridges. It is also the intention that this modification should improve the durability of a pharmaceutical composition which can be employed in one of the uses according to the invention. In general, the oligonucleotide can be modified at the base moiety, sugar moiety, or phosphate backbone.

The oligonucleotide may include other appending groups such as peptides, agents facilitating transport across the cell membrane (see, e.g., Letsinger et al., 1989, Proc. Natl. Acad. Sci. USA 86:6553-6556; Lemaitre et al., 1987, Proc. Natl. Acad. Sci. USA 84:648-652; International Patent Publication No. WO 88/09810) or blood-brain barrier (see, e.g., International Patent Publication No. WO 89/10134), hybridization-triggered cleavage agents (see, e.g., Krol et al., 1988, BioTechniques 6:958-976), or intercalating agents (see, e.g., Zon, 1988, Pharm. Res. 5:539-549).

In detail, the antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thio-uridine, 5-carboxymethylaminomethyluracil, dihydrouracil, D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, D-mannosylqueosine, 5N-methoxycarboxymethyluracil, 5-methoxyuracil, 2-methyl-thio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

The use of suitable antisense nucleic acids is further described e.g. in Zheng and Kemeny (1995) Clin. Exp. Immunol., 100, 380-2; Nellen and Lichtenstein (1993) Trends Biochem. Sci., 18, 419-23, Stein (1992) Leukemia, 6, 697-74 or Yacyshyn, B. R. et al. (1998) Gastroenterology, 114, 1142).

In yet another embodiment, the oligonucleotide is a 2-a-anomeric oligonucleotide. An a-anomeric oligonucleotide (2-a-anomeric oder a-anomeric) forms specific double-stranded hybrids with complementary RNA in which, contrary to the usual B-units, the strands run parallel to each other (Gautier et al., 1987, Nucl. Acids Res. 15:6625-6641).

The oligonucleotide may be conjugated to another molecule, e.g., a peptide, hybridization-triggered cross-linking agent, transport agent, hybridization-triggered cleavage agent, etc.

Throughout the invention, oligonucleotides of the invention may be synthesized by standard methods known in the art, e.g., by use of an automated DNA synthesizer (such as are commercially available from Biosearch, Applied Biosystems, etc.). As examples, phosphorothioate oligo-nucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. USA 85:7448-7451), etc.

In a specific embodiment, the antisense oligonucleotides comprise catalytic RNAs, or ribozymes (see, e.g., International Patent Publication No. WO 90/11364; Sarver et al., 1990, Science 247:1222-1225). In another embodiment, the oligonucleotide is a 2'-0-methylribonucleotide (Inoue et al., 1987, Nucl. Acids Res. 15:6131-6148), or a chimeric RNA-DNA analog (Inoue et al., 1987, FEBS Lett. 215:327-330).

In an alternative embodiment, the antisense nucleic acids of the invention are produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding the component protein. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art to be capable of replication and expression in mammalian cells. Expression of the sequences encoding the antisense RNAs can be by any promoter known in the art to act in mammalian, preferably human, cells. Such promoters can be inducible or constitutive. Such promoters include, but are not limited to, the SV40 early promoter region (Bernoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. USA 78:1441-1445), the regulatory sequences of the metallothionein gene (Brinster et al., 1982, Nature 296:39-42), etc.

The antisense nucleic acids of the invention comprise a sequence complementary to at least a portion of an RNA transcript of a component protein gene, preferably a human gene. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a component protein RNA it may contain and still form a stable duplex (or triplex, as the case may be). One skilled in the art can ascertain a tolerable degree of mismatch by use of standard procedures to determine the melting point of the hybridized complex.

The production and use of siRNAs as tools for RNA interference in the process to down regulate or to switch off gene expression, here GPR49 gene expression, is e.g. described in Elbashir, S. M. et al. (2001) Genes Dev., 15, 188 or Elbashir, S. M. et al. (2001) Nature, 411, 494. Preferably, siRNAs exhibit a length of less than 30 nucleotides, wherein the identity stretch of the sense strang of the siRNA is preferably at least 19 nucleotides.

Ribozymes are also suitable tools to inhibit the translation of nucleic acids, here the GPR49 gene, because they are able to specifically bind and cut the mRNAs. They are e.g. described in Amarzguioui et al. (1998) Cell. Mol. Life Sci., 54, 1175-202; Vaish et al. (1998) Nucleic Acids Res., 26, 5237-42; Persidis (1997) Nat. Biotechnol., 15, 921-2 or Couture and Stinchcomb (1996) Trends Genet., 12, 510-5.

Pharmaceutical compositions of the invention, comprising an effective amount of a nucleic acid in a pharmaceutically acceptable carrier, can be administered to a patient having a disease or disorder that is of a type that expresses or overexpresses a protein complex of the present invention.

The amount of the nucleic acid that will be effective in the treatment of a particular disorder or condition will depend on the nature of the disorder or condition, and can be determined by standard clinical techniques. Where possible, it is desirable to determine the nucleic acid cytotoxicity in vitro, and then in useful animal model systems, prior to testing and use in humans.

In a specific embodiment, pharmaceutical compositions comprising nucleic acids are administered via liposomes, microparticles, or microcapsules. In various embodiments of the invention, it may be useful to use such compositions to achieve sustained release of the nucleic acids. In a specific embodiment, it may be desirable to utilize liposomes targeted via antibodies to specific identifiable central nervous system cell types (Leonetti et al., 1990, Proc. Natl. Acad. Sci. U.S.A. 87:2448-2451; Renneisen et al., 1990, J. Biol. Chem. 265:16337-16342).

So-called "low molecular weight molecules" (in the following called "LMWs") are molecules which are not proteins, peptides, antibodies or nucleic acids, and which exhibit a molecular weight of less than 5000 Da, preferably less than 2000 Da, more preferably less than 1000 Da, most preferably less than 500 Da. Such LMWs may be identified in high-throughput procedures starting from libraries. Such methods are known in the art and are discussed in detail below.

The term "binding protein" or "binding peptide" refers to a class of proteins or peptides which bind and inhibit GPR49, and includes, without limitation, polyclonal or monoclonal antibodies, antibody fragments and protein scaffolds directed against GPR49.

According to the present invention, the term antibody or antibody fragment is also understood as meaning antibodies or antigen-binding parts thereof, which have been prepared recombinantly and, where appropriate, modified, such as chimeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884), preferably produced with the help of a FAB expression library.

As an alternative to the classical antibodies it is also possible, for example, to use protein scaffolds against GPR49, e.g. anticalins which are based on lipocalin (Beste et al. (1999) Proc. Natl. Acad. Sci. USA, 96, 1898-1903). The natural ligand-binding sites of the lipocalins, for example the retinol-binding protein or the bilin-binding protein, can be altered, for example by means of a "combinatorial protein design" approach, in such a way that they bind to selected haptens, here to GPR49 (Skerra, 2000, Biochim. Biophys. Acta, 1482, 337-50). Other known protein scaffolds are known as being alternatives to antibodies for molecular recognition (Skerra (2000) J. Mol. Recognit., 13, 167-187).

The procedure for preparing an antibody or antibody fragment is effected in accordance with methods which are well known to the skilled person, e.g. by immunizing a mammal, for example a rabbit, with GPR49, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromatography. Monoclonal antibodies can, for example, be prepared in accordance with the known method of Winter & Milstein (Winter, G. & Milstein, C. (1991) Nature, 349, 293-299).

In detail, polyclonal antibodies can be prepared as described above by immunizing a suitable subject with a polypeptide as an immunogen. Preferred polyclonal antibody compositions are ones that have been selected for antibodies directed against a polypeptide or polypeptides of the invention. Particularly preferred polyclonal antibody preparations are ones that contain only antibodies directed against a given polypeptide or polypeptides. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. Alternatively, antibodies specific for a protein or polypeptide of the invention can be selected for (e.g., partially purified) or purified by, e.g., affinity chromatography. For example, a recombinantly expressed and purified (or partially purified) protein of the invention is produced as described herein, and covalently or non-covalently coupled to a solid support such as, for example, a chromatography column. The column can then be used to affinity purify antibodies specific for the proteins of the invention from a sample containing antibodies directed against a large number of different epitopes, thereby generating a substantially purified antibody composition, i.e., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein, 1975, Nature 256:495-497, the human B cell hybridoma technique (Kozbor et al., 1983, Immunol. Today 4:72), the EBV-hybridoma technique (Cole et al., 1985, Monoclonal Antibodies and Cancer Therapy, Alan R. Liss, Inc., pp. 77-96) or trioma techniques. The technology for producing hybridomas is well known (see generally Current Protocols in Immunology 1994, Coligan et al. (eds.) John Wiley & Sons, Inc., New York, NY). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., 1991, Bio/Technology 9:1370-1372; Hay et al., 1992, Hum. Antibod. Hybridomas 3:81-85; Huse et al., 1989, Science 246:1275-1281; Griffiths et al., 1993, EMBO J. 12:725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, e.g., Cabilly et al., U.S. Patent No. 4,816,567; and Boss et al., U.S. Patent No. 4,816,397, which are incorporated herein by reference in their entirety.) Humanized antibodies are antibody molecules from non-human species having one or more complementarily determining regions (CDRs) from the non-human species and a framework region from a human immunoglobulin molecule. (See, e.g., Queen, U.S. Patent No. 5,585,089, which is incorporated herein by reference in its entirety.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559); Morrison, 1985, Science 229:1202-1207; Oi et al., 1986, Bio/Techniques 4:214; U.S. Patent 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar, 1995, Int. Rev. Immunol. 13:65-93). For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope. (Jespers et al., 1994, Bio/technology 12:899-903).

Antibody fragments that contain the idiotypes of the complex can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments.

In the production of antibodies, screening for the desired antibody can be accomplished by techniques known in the art, e.g., ELISA (enzyme-linked immunosorbent assay). To select antibodies specific to a particular domain of the complex, or a derivative thereof, one may assay generated hybridomas for a product that binds to the fragment of the complex, or a derivative thereof, that contains such a domain. For selection of an antibody that specifically binds a complex of the present, or a derivative, or homologue thereof, but which does not specifically bind to the individual proteins of the complex, or a derivative, or homologue thereof, one can select on the basis of positive binding to the complex and a lack of binding to the individual protein components.

The foregoing antibodies can be used in methods known in the art relating to the localization and/or quantification of the given protein or proteins, e.g., for imaging these proteins, measuring levels thereof in appropriate physiological samples (by immunoassay), in diagnostic methods, etc. This hold true also for a derivative, or homologue thereof of a complex.

In a preferred embodiment, the GPR49-inhibitor is an siRNA with the sequence:
AACAGCAGTATGGACGACCTT

GPR49 shows similarity to receptors of LH (lueteinizing hormone), relaxin, IGF, FSH (follicle stimulating hormone), and TSH. Therefore, LH, relaxin, IGF, FSH, and TSH could be physiological ligands of GPR49. Furthermore, thieno[2,3-d]pyrimidines identified as LH and FSH receptor agonists
in WO-03 020 726 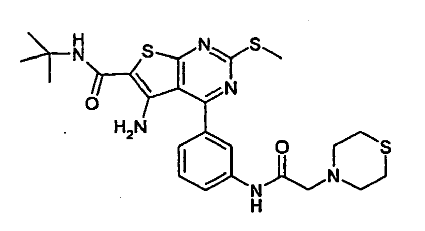 and WO-00 187 287 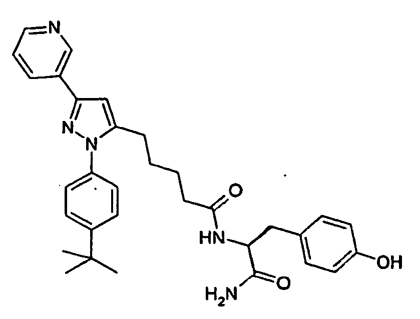 may be agonists of GPR49.

As discussed above, GPR49 is part of protein complexes which are involved in the regulation of gamma secretase activity and/or beta-secretase. Therefore, in a preferred embodiment, the GPR49 interacting molecule or inhibitor acts on a GPR49 molecule which is part of a protein complex, preferably of the Aphla-complex, of the Fe65L2-complex, of the APP-C99-complex, or of the BACE1-complex.

Said protein complexes have been identified as assemblies of proteins interacting with the gamma-secretase subunit Aph1 a the gamma-secretase substrate APP-C99 (plus its adapter Fe65L2) and with beta-secretase protein.

Presenilins 1 and 2 (Psen1 and Psen2, also referred to as PS 1 and PS2 respectively) are integral membrane proteins which are localised in the endoplasmic reticulum, the Golgi and also at the cell surface (Kovacs, Nat Med 2. 224). They are predominantly found as a heterodimers of the NTF and CTF endoproteolytic fragments. The protease that cleaves presenilins (the "presenilinase") is not known, it is likely that the process is autocatalytic, also the functional significance of PS (auto)proteolysis is unclear. Presenilins are involved in the proteolytical processing of Amyloid precursor protein (APP) (De Strooper et al, Nature 391, 387) and the Notch receptor (De Strooper et al. Nature 398, 518). In addition, Presenilins are associated with the cell-adhesion proteins alpha and beta-catenin, N-cadherin, and E-cadherin (Georgakopoulos et al, Mol Cell 4, 893) and other members of the armadillo family (Yu et al, J Biol Chem 273, 16470). APP processing by Presenilins is through their effects on gamma-secretase which cleaves APP, generating the C-terminus of the A-beta peptide. PS1 associates with the C83 and C99 processed C-terminal fragments of APP (Xia et al, Proc Natl Acad Sci USA, 94, 8208), Nicastrin (Yu et al, Nature 407, 48) and Pen-2 (Francis et al, Dev Cell 3, 85).

Aph-1 proteins such as Aph-1a are (Francis et al, Dev Cell 3, 85) required in Presenilin processing. It is not clear whether Presenilins regulate gamma-secretase activity directly or whether they are protease enzymes themselves (Kopan and Gouate, Genes Dev 14, 2799). The gamma secretase activity could comprise a multimeric complex of these proteins (Yu et al, Nature 407, 48) but it is not known how the relationship between these proteins affects secretase activity.

Aph-1 and Pen-2 were cloned recently in a screen for presenilin enhancers ("pen") in C. elegans and shown to interact genetically with Aph-2 (Nicastrin). Defects in Aph-1 affect Notch signalling and Nicastrin localization. Aph-1 and Pen-2 are required for Notch cleavage, gamma-secretase activity and the accumulation of processed Presenilins. Francis et al. cloned the putative human orthologues of these genes, Aph-1a, Aph-1b and Pen-2, and recently Lee et al. also cloned the human Aph-1 cDNAs. The exact components of the gamma-secretase complex are not known but these two novel proteins could be components of or accessory factors to the complex and may interact together directly with Presenilin or with a Presenilin/Nicastrin complex. Nicastrin is therefore a member of the active gamma-secretase complex and there is recent evidence that it is the fully glycosylated form of the protein which is important in this complex.

Fe65-like 2 (Fe65L2) is a homolog of the well-characterized intracellular APP-interacting adapter protein Fe65 (Duilio A, Faraonio R, Minopoli G, Zambrano N, Russo T (1998) Fe65L2: a new member of the Fe65 protein family interacting with the intracellular domain of the Alzheimer's beta-amyloid precursor protein. Biochem J. 330 (Pt 1):513-9.). It is highly expressed in brain and interacts with APP through its C-terminal PTB domain (Tanahashi H, Tabira T (1999) Molecular cloning of human Fe65L2 and its interaction with the Alzheimer's beta-amyloid precursor protein. Neurosci Lett. 261 (3):143-6.). Fe65L2 has been shown to translocate to the nucleus and regulate transcription (Bruni P, Minopoli G, Brancaccio T, Napolitano M, Faraonio R, Zambrano N, Hansen U, Russo T (2002) Fe65, a ligand of the Alzheimer's beta-amyloid precursor protein, blocks cell cycle progression by down-regulating thymidylate synthase expression. J. Biol. Chem. 277(38): 35481-8.) suggesting that it be involved in down-stream signalling functions of APP. Overexpression of Fe65L2 promotes secretion/generation of Abeta in vitro (Tanahashi H, Tabira T (2002) Characterization of an amyloid precursor protein-binding protein Fe65L2 and its novel isoforms lacking phosphotyrosine-interaction domains. Biochem J. 367(Pt 3):687-95.).

The beta-secretase (BACE) activity cleaves APP in the ectodomain, resulting in shedding of secreted, soluble APPb, and in a 99-residue C-terminal transmembrane fragment (APP-C99). Vassar et al. (Science 286, 735-741) cloned a transmembrane aspartic protease that had the characteristics of the postulated beta-secretase of APP, which they termed BACE1. Brain and primary cortical cultures from BACE1 knockout mice showed no detectable beta-secretase activity, and primary cortical cultures from BACE knockout mice produced much less amyloid-beta from APP. This suggests that BACE1, rather than its paralogue BACE2, is the main beta-secretase for APP. BACE1 is a protein of 501 amino acids containing a 21-aa signal peptide followed by a proprotein domain spanning aa 22 to 45. There are alternatively spliced forms, BACE-I-457 and BACE-I-476. The lumenal domain of the mature protein is followed by one predicted transmembrane domain and a short cytosolic C-terminal tail of 24 aa. BACE1 is predicted to be a type 1 transmembrane protein with the active site on the lumenal side of the membrane, where beta-secretase cleaves APP and possible other yet unidentified substrates. Although BACE1 is clearly a key enzyme required for the processing of APP into A-beta, recent evidence suggests additional potential substrates and functions of BACE1 (J. Biol. Chem. 279, 10542-10550). To date, no BACE1 interacting proteins with regulatory or modulatory functions have been described.

As explained above, it has been surprisingly found in the context of the present invention that GPR49 is part of the protein complexes regulating proteolytic processing of APP, in particular by beta-secretase and/or gamma secretase activity. Therefore, in a preferred embodiment, the inhibitor or interacting molecule modulates the activity of beta-secretase and/or gamma secretase.

Throughout the invention, the term "modulating the activity of gamma secretase and/or beta secretase" includes that the activity of the enzyme is modulated directly or indirectly. That means that the GPR49 modulator may either bind also directly to either of these enzymes or, more preferred, may exert an influence on GPR49 which in turn, e.g. by protein-protein interactions or by signal transduction or via small metabolites, modulates the activity of either of these enzymes.

Throughout the invention, it is preferred that the beta secretase modulator inhibits the activity of beta secretase either completely or partially. Throughout the invention, the most preferred functional consequence of a GPR49 modulator is a reduction in Abeta-42 generation.

In the context of the present invention, "modulating the activity of gamma secretase and/or beta secretase" means that the activity is reduced in that less or no product is formed, most preferably that less or no Abeta-42 is formed, (partial or complete inhibition) or that the respective enzyme produces a different product (in the case of gamma-secretase e.g. Abeta-38 or other Abeta peptide species of shorter amino acid sequence - instead of Abeta-42) or that the relative quantities of the products are different (in the case of gamma-secretase e.g. the ratio of Abeta-40 to Abeta-42 is changed preferably increased). Furthermore, it is included that the modulator modulates either gamma secretase or beta-secretase or the activity of both enzymes.

With respect to the modulator of gamma secretase activity, it is preferred that this modulator inhibits gamma secretase activity. However, it is also preferred that the activity of gamma secretase is shifted in a way that the total amount of Abeta peptide species is unchanged but that more Abeta-38 is produced instead of Abeta-42.

Gamma secretase activity can e.g. measured by determining APP processing, e.g. by determining levels of Abeta peptide species produced, most importantly levels of Abeta-42 (see Example-section, infra).

To measure BACE1 activity, changes of the ratio between alpha- and beta-C-terminal APP fragments can be analyzed by Western Blotting (Blasko et al., J Neural Transm 111, 523); additional examples for BACE1 activity assays include but are not limited to: use of a cyclized enzyme donor peptide containing a BACE1 cleavage site to reconstitute and measure beta-galactosidase reporter activity (Naqvi et al., J Biomol Screen. 9, 398); use of quenched fluorimetric peptide substrates and fluorescence measurements (Andrau et al., J.Biol Chem 278, 25859); use of cell-based assays utilizing recombinant chimeric proteins, in which an enzyme (such as alkaline phosphatase) is linked via a stretch of amino acids, that contain the BACE1 recognition sequence, to a Golgi-resident protein (Oh et al., Anal Biochem, 323, 7); fluorescence resonance energy transfer (FRET)-based assays (Kennedy et al., Anal Biochen 319, 49); a cellular growth selection system in yeast (Luthi et al., Biochim Biophys Acta 1620, 167).

Preferably, the neurodegenerative disease is Alzheimer's disease.

According to the invention, the GPR49 inhibitor is used to prepare a pharmaceutical composition.

Therefore, the invention provides pharmaceutical compositions, which may be administered to a subject in an effective amount. In a preferred aspect, the therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the therapeutic can be delivered via a controlled release system. In one embodiment, a pump may be used (Langer, supra; Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:85 8-863). In yet another embodiment, a controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In a specific embodiment where the therapeutic is a nucleic acid, preferably encoding a protein therapeutic, the nucleic acid can be administered in vivo to promote expression of its encoded protein, by constructing it as part of an appropriate nucleic acid expression vector and administering it so that it becomes intracellular, e.g., by use of a retroviral vector (U.S. Patent No. 4,980,286), or by direct injection, or by use of microparticle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864-1868), etc. Alternatively, a nucleic acid therapeutic can be introduced intracellularly and incorporated by homologous recombination within host cell DNA for expression.

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits of the present invention can also contain expression vectors encoding the essential components of the complex machinery, which components after being expressed can be reconstituted in order to form a biologically active complex. Such a kit preferably also contains the required buffers and reagents. Optionally associated with such container(s) can be instructions for use of the kit and/or a notice in the form prescribed by a governmental agency regulating the manufacture, use or sale of pharmaceuticals or biological products, which notice reflects approval by the agency of manufacture, use or sale for human administration.

The invention further relates to a method of treatment, wherein an effective amount of a GPR49-interacting molecule or inhibitor or of a pharmaceutical composition of the invention is administered to a subject suffering from a neurodegenerative disease, preferably Alzheimer's disease.

With respect to this method of the invention, all embodiments apply given above for the use of the invention.

The invention further relates to a method for identifying a gamma secretase modulator and/or beta-secretase modulator, comprising the following steps:
a. identifying of a GPR49-interacting molecule by determining whether a given test compound is a GPR49-interacting molecule,
b. determining whether the GPR49-interacting molecule of step a) is capable of modulating gamma secretase activity or beta-secretase activity.

In a preferred embodiment of the invention, in step a) the test compound is brought into contact with GPR49 and the interaction of GPR49 with the test compound is determined. Preferably, it is measured whether the candidate molecule is bound to GPR49.

In a preferred embodiment of the invention, the GPR49 interacting molecule identified in step a) is first subjected to a GPR49 activity test as decribed supra (also see example 3) in order to find out whether it modulates, preferably inhibits GPR49 activity and is then subjected to process step b) (test for a Abeta-lowering effect).

The method of the invention is preferably performed in the context of a high throughput assay. Such assays are known to the person skilled in the art.

Test or candidate molecules to be screened can be provided as mixtures of a limited number of specified compounds, or as compound libraries, peptide libraries and the like. Agents/molecules to be screened may also include all forms of antisera, antisense nucleic acids, etc., that can modulate complex activity or formation. Exemplary candidate molecules and libraries for screening are set forth below.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with a GPR49 immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

In a specific embodiment, GPR49-fragments and/or analogs, especially peptidomimetics, are screened for activity as competitive or non-competitive inhibitors of the formation of a complex of GPR49 with another proteins, e.g. the proteins given in Table 1 (amount of complex or composition of complex) or GPR49 activity in the cell, which thereby inhibit complex activity or formation in the cell.

In one embodiment, agents that modulate (i.e., antagonize or agonize) GPR49-activity or GPR49-protein complex formation can be screened for using a binding inhibition assay, wherein agents are screened for their ability to modulate formation of a complex under aqueous, or physiological, binding conditions in which complex formation occurs in the absence of the agent to be tested. Agents that interfere with the formation of complexes of the invention are identified as antagonists of complex formation. Agents that promote the formation of complexes are identified as agonists of complex formation. Agents that completely block the formation of complexes are identified as inhibitors of complex formation.

Methods for screening may involve labeling the component proteins of the complex with radioligands (e.g., ¹²⁵I or ³H), magnetic ligands (e.g., paramagnetic beads covalently attached to photobiotin acetate), fluorescent ligands (e.g., fluorescein or rhodamine), or enzyme ligands (e.g., luciferase or β-galactosidase). The reactants that bind in solution can then be isolated by one of many techniques known in the art, including but not restricted to, co-immunoprecipitation of the labeled complex moiety using antisera against the unlabeled binding partner (or labeled binding partner with a distinguishable marker from that used on the second labeled complex moiety), immunoaffinity chromatography, size exclusion chromatography, and gradient density centrifugation. In a preferred embodiment, the labeled binding partner is a small fragment or peptidomimetic that is not retained by a commercially available filter. Upon binding, the labeled species is then unable to pass through the filter, providing for a simple assay of complex formation.

Methods commonly known in the art are used to label at least one of the component members of the complex. Suitable labeling methods include, but are not limited to, radiolabeling by incorporation of radiolabeled amino acids, e.g., ³H-leucine or ³⁵S-methionine, radiolabeling by post-translational iodination with ¹²⁵I or ¹³¹I using the chloramine T method, Bolton-Hunter reagents, etc., or labeling with ³²P using phosphorylase and inorganic radiolabeled phosphorous, biotin labeling with photobiotin-acetate and sunlamp exposure, etc. In cases where one of the members of the complex is immobilized, e.g., as described infra, the free species is labeled. Where neither of the interacting species is immobilized, each can be labeled with a distinguishable marker such that isolation of both moieties can be followed to provide for more accurate quantification, and to distinguish the formation of homomeric from heteromeric complexes. Methods that utilize accessory proteins that bind to one of the modified interactants to improve the sensitivity of detection, increase the stability of the complex, etc., are provided.

Typical binding conditions are, for example, but not by way of limitation, in an aqueous salt solution of 10-250 mM NaCl, 5-50 mM Tris-HCl, pH 5-8, and 0.5% Triton X-100 or other detergent that improves specificity of interaction. Metal chelators and/or divalent cations may be added to improve binding and/or reduce proteolysis. Reaction temperatures may include 4, 10, 15, 22, 25, 35, or 42 degrees Celsius, and time of incubation is typically at least 15 seconds, but longer times are preferred to allow binding equilibrium to occur. Particular complexes can be assayed using routine protein binding assays to determine optimal binding conditions for reproducible binding.

The physical parameters of complex formation can be analyzed by quantification of complex formation using assay methods specific for the label used, e.g., liquid scintillation counting for radioactivity detection, enzyme activity for enzyme-labeled moieties, etc. The reaction results are then analyzed utilizing Scatchard analysis, Hill analysis, and other methods commonly known in the arts (see, e.g., Proteins, Structures, and Molecular Principles, 2nd Edition (1993) Creighton, Ed., W.H. Freeman and Company, New York).

In a second common approach to binding assays, one of the binding species is immobilized on a filter, in a microtiter plate well, in a test tube, to a chromatography matrix, etc., either covalently or non-covalently. Proteins can be covalently immobilized using any method well known in the art, for example, but not limited to the method of Kadonaga and Tjian, 1986, Proc. Nat1. Acad. Sci. USA 83:5889-5893, i.e., linkage to a cyanogen-bromide derivatized substrate such as CNBr-Sepharose 4B (Pharmacia). Where needed, the use of spacers can reduce steric hindrance by the substrate. Non-covalent attachment of proteins to a substrate include, but are not limited to, attachment of a protein to a charged surface, binding with specific antibodies, binding to a third unrelated interacting protein, etc.

Assays of agents (including cell extracts or a library pool) for competition for binding of one member of a complex (or derivatives thereof) with another member of the complex labeled by any means (e.g., those means described above) are provided to screen for competitors or enhancers of complex formation.

In specific embodiments, blocking agents to inhibit non-specific binding of reagents to other protein components, or absorptive losses of reagents to plastics, immobilization matrices, etc., are included in the assay mixture. Blocking agents include, but are not restricted to bovine serum albumin, casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow complex formation.

After binding is performed, unbound, labeled protein is removed in the supernatant, and the immobilized protein retaining any bound, labeled protein is washed extensively. The amount of bound label is then quantified using standard methods in the art to detect the label as described, supra.

In another specific embodiments screening for modulators of the protein complexes/protein as provided herein can be carried out by attaching those and/or the antibodies as provided herein to a solid carrier.

The preparation of such an array containing different types of proteins, including antibodies) is well known in the art and is apparent to a person skilled in the art (see e.g. Ekins et al., 1989, J. Pharm. Biomed. Anal. 7:155-168; Mitchell et al. 2002, Nature Biotechnol. 20:225-229; Petricoin et al., 2002, Lancet 359:572-577; Templin et al., 2001, Trends Biotechnol. 20:160-166; Wilson and Nock, 2001, Curr. Opin. Chem. Biol. 6:81-85; Lee et al., 2002 Science 295:1702-1705; MacBeath and Schreiber, 2000, Science 289:1760; Blawas and Reichert, 1998, Biomaterials 19:595; Kane et al., 1999, Biomaterials 20:2363; Chen et al., 1997, Science 276:1425; Vaugham et al., 1996, Nature Biotechnol. 14:309-314; Mahler et al., 1997, Immunotechnology 3:31-43; Roberts et al., 1999, Curr. Opin. Chem. Biol. 3:268-273; Nord et al., 1997, Nature Biotechnol. 15:772-777; Nord et al., 2001, Eur. J. Biochem. 268:4269-4277; Brody and Gold, 2000, Rev. Mol. Biotechnol. 74:5-13; Karlstroem and Nygren, 2001, Anal. Biochem. 295:22-30; Nelson et al., 2000, Electrophoresis 21:1155-1163; Honore et al., 2001, Expert Rev. Mol. Diagn. 3:265-274; Albala, 2001, Expert Rev. Mol. Diagn. 2:145-152, Figeys and Pinto, 2001, Electrophoresis 2:208-216 and references in the publications listed here).

Protein or protein complexes can be attached to an array by different means as will be apparent to a person skilled in the art. Complexes can for example be added to the array via a TAP-tag (as described in WO/0009716 and in Rigaut et al., 1999, Nature Biotechnol. 10:1030-1032) after the purification step or by another suitable purification scheme as will be apparent to a person skilled in the art.

Optionally, the proteins of the complex can be cross-linked to enhance the stability of the complex. Different methods to cross-link proteins are well known in the art. Reactive endgroups of cross-linking agents include but are not limited to -COOH, -SH, -NH2 or N-oxy-succinamate.

The spacer of the cross-linking agent should be chosen with respect to the size of the complex to be cross-linked. For small protein complexes, comprising only a few proteins, relatively short spacers are preferable in order to reduce the likelihood of cross-linking separate complexes in the reaction mixture. For larger protein complexes, additional use of larger spacers is preferable in order to facilitate cross-linking between proteins within the complex.

It is preferable to check the success-rate of cross-linking before linking the complex to the carrier.

As will be apparent to a person skilled in the art, the optimal rate of cross-linking need to be determined on a case by case basis. This can be achieved by methods well known in the art, some of which are exemplary described below.

A sufficient rate of cross-linking can be checked f.e. by analysing the cross-linked complex vs. a non-cross-linked complex on a denaturating protein gel.

If cross-linking has been performed successfully, the proteins of the complex are expected to be found in the same lane, whereas the proteins of the non-cross-linked complex are expected to be separated according to their individual characteristics. Optionally the presence of all proteins of the complex can be further checked by peptide-sequencing of proteins in the respective bands using methods well known in the art such as mass spectrometry and/or Edman degradation.

In addition, a rate of crosslinking which is too high should also be avoided. If cross-linking has been carried out too extensively, there will be an increasing amount of cross-linking of the individual protein complex, which potentially interferes with a screening for potential binding partners and/or modulators etc. using the arrays.

The presence of such structures can be determined by methods well known in the art and include e.g. gel-filtration experiments comparing the gel filtration profile solutions containing cross-linked complexes vs. uncross-linked complexes.

Optionally, functional assays as will be apparent to a person skilled in the art, some of which are exemplarily provided herein, can be performed to check the integrity of the complex.

Alternatively, the proteins or the protein can be expressed as a single fusion protein and coupled to the matrix as will be apparent to a person skilled in the art.

Optionally, the attachment of the complex or proteins or antibody as outlined above can be further monitored by various methods apparent to a person skilled in the art. Those include, but are not limited to surface plasmon resonance (see e.g. McDonnel, 2001, Curr. Opin. Chem. Biol. 5:572-577; Lee, 2001, Trends Biotechnol. 19:217-222; Weinberger et al., 2000, 1:395-416; Pearson et al., 2000, Ann. Clin. Biochem. 37:119-145; Vely et al., 2000, Methods Mol. Biol. 121:313-321; Slepak, 2000, J. Mol Recognit. 13:20-26.

Exemplary assays useful for measuring the production of Abeta-40 and Abeta-42 peptides by ELISA include but are not limited to those described in Vassar R et al., 1999, Science, 286:735-41.

Exemplary assays useful for measuring the production of C-terminal APP fragments in cell lines or transgenic animals by western blot include but are not limited to those described in Yan R et al., 1999, Nature, 402:533-7.

Exemplary assays useful for measuring the proteolytic activity of beta- or gamma secretases towards bacterially expressed APP fragments in vitro (e.g. by modifying the expression of one or several interacting proteins in cells by means of RNAi (siRNA) and/or plasmids encoding the interacting protein(s)) of the BACE1-complex include but are not limited to those described in Tian G et al., 2002, J Biol Chem, 277:31499-505.

Exemplary assays useful for measuring transactivation of a Gal4-driven reporter gene (e.g. by modifying the expression of one or several interacting proteins in cells by means of RNAi (siRNA) and/or plasmids encoding the interacting protein(s)) of the BACE1-complex include but are not limited to those described in Cao X et al., 2001, Science, 293:115-20.

Any molecule known in the art can be tested for its ability to be an interacting molecule or inhibitor according to the present invention. Candidate molecules can be directly provided to a cell expressing the GPR49-complex machinery, or, in the case of candidate proteins, can be provided by providing their encoding nucleic acids under conditions in which the nucleic acids are recombinantly expressed to produce the candidate protein.

The method of the invention is well suited to screen chemical libraries for molecules which modulate, e.g., inhibit, antagonize, or agonize, the amount of, activity of, or protein component composition of the complex. The chemical libraries can be peptide libraries, peptidomimetic libraries, chemically synthesized libraries, recombinant, e.g., phage display libraries, and in vitro translation-based libraries, other non-peptide synthetic organic libraries, etc.

Exemplary libraries are commercially available from several sources (ArQule, Tripos/PanLabs, ChemDesign, Pharmacopoeia). In some cases, these chemical libraries are generated using combinatorial strategies that encode the identity of each member of the library on a substrate to which the member compound is attached, thus allowing direct and immediate identification of a molecule that is an effective modulator. Thus, in many combinatorial approaches, the position on a plate of a compound specifies that compound's composition. Also, in one example, a single plate position may have from 1-20 chemicals that can be screened by administration to a well containing the interactions of interest. Thus, if modulation is detected, smaller and smaller pools of interacting pairs can be assayed for the modulation activity. By such methods, many candidate molecules can be screened.

Many diversity libraries suitable for use are known in the art and can be used to provide compounds to be tested according to the present invention. Alternatively, libraries can be constructed using standard methods. Chemical (synthetic) libraries, recombinant expression libraries, or polysome-based libraries are exemplary types of libraries that can be used.

The libraries can be constrained or semirigid (having some degree of structural rigidity), or linear or nonconstrained. The library can be a cDNA or genomic expression library, random peptide expression library or a chemically synthesized random peptide library, or non-peptide library. Expression libraries are introduced into the cells in which the assay occurs, where the nucleic acids of the library are expressed to produce their encoded proteins.

In one embodiment, peptide libraries that can be used in the present invention may be libraries that are chemically synthesized in vitro. Examples of such libraries are given in Houghten et al., 1991, Nature 354:84-86, which describes mixtures of free hexapeptides in which the first and second residues in each peptide were individually and specifically defined; Lam et al., 1991, Nature 354:82-84, which describes a "one bead, one peptide" approach in which a solid phase split synthesis scheme produced a library of peptides in which each bead in the collection had immobilized thereon a single, random sequence of amino acid residues; Medynski, 1994, Bio/Technology 12:709-710, which describes split synthesis and T-bag synthesis methods; and Gallop et al., 1994, J. Med. Chem. 37:1233-1251. Simply by way of other examples, a combinatorial library may be prepared for use, according to the methods of Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; or Salmon et al., 1993, Proc. Natl. Acad. Sci. USA 90:11708-11712. PCT Publication No. WO 93/20242 and Brenner and Lerner, 1992, Proc. Natl. Acad. Sci. USA 89:5381-5383 describe "encoded combinatorial chemical libraries," that contain oligonucleotide identifiers for each chemical polymer library member.

In a preferred embodiment, the library screened is a biological expression library that is a random peptide phage display library, where the random peptides are constrained (e.g., by virtue of having disulfide bonding).

Further, more general, structurally constrained, organic diversity (e.g., nonpeptide) libraries, can also be used. By way of example, a benzodiazepine library (see e.g., Bunin et al., 1994, Proc. Natl. Acad. Sci. USA 91:4708-4712) may be used.

Conformationally constrained libraries that can be used include but are not limited to those containing invariant cysteine residues which, in an oxidizing environment, cross-link by disulfide bonds to form cystines, modified peptides (e.g., incorporating fluorine, metals, isotopic labels, are phosphorylated, etc.), peptides containing one or more non-naturally occurring amino acids, non-peptide structures, and peptides containing a significant fraction of -carboxyglutamic acid.

Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the alpha-carbon but to the backbone amino nitrogen. Since peptoids are not easily degraded by human digestive enzymes, they are advantageously more easily adaptable to drug use. Another example of a library that can be used, in which the amide functionalities in peptides have been permethylated to generate a chemically transformed combinatorial library, is described by Ostresh et al., 1994, Proc. Natl. Acad. Sci. USA 91:11138-11142).

The members of the peptide libraries that can be screened according to the invention are not limited to containing the 20 naturally occurring amino acids. In particular, chemically synthesized libraries and polysome based libraries allow the use of amino acids in addition to the 20 naturally occurring amino acids (by their inclusion in the precursor pool of amino acids used in library production). In specific embodiments, the library members contain one or more non-natural or non-classical amino acids or cyclic peptides. Non-classical amino acids include but are not limited to the D-isomers of the common amino acids, - amino isobutyric acid, 4-aminobutyric acid, Abu, 2-amino butyric acid; -Abu, -Ahx, 6-amino hexanoic acid; Aib, 2-amino isobutyric acid; 3-amino propionic acid; omithine; norleucine; norvaline, hydroxyproline, sarcosine, citrulline, cysteic acid, t-butylglycine, t-butylalanine, phenylglycine, cyclohexylalanine, B-alanine, designer amino acids such as B-methyl amino acids, C- methyl amino acids, N- methyl amino acids, fluoro-amino acids and amino acid analogs in general. Furthermore, the amino acid can be D (dextrorotary) or L (levorotary).

In a specific embodiment, fragments and/or analogs of complexes of the invention, or protein components thereof, especially peptidomimetics, are screened for activity as competitive or non-competitive inhibitors of complex activity or formation.

In another embodiment of the present invention, combinatorial chemistry can be used to identify modulators of a the complexes. Combinatorial chemistry is capable of creating libraries containing hundreds of thousands of compounds, many of which may be structurally similar. While high throughput screening programs are capable of screening these vast libraries for affinity for known targets, new approaches have been developed that achieve libraries of smaller dimension but which provide maximum chemical diversity. (See e.g., Matter, 1997, J. Med. Chem. 40:1219-1229).

One method of combinatorial chemistry, affinity fingerprinting, has previously been used to test a discrete library of small molecules for binding affinities for a defined panel of proteins. The fingerprints obtained by the screen are used to predict the affinity of the individual library members for other proteins or receptors of interest (in the instant invention, the protein complexes of the present invention and protein components thereof.) The fingerprints are compared with fingerprints obtained from other compounds known to react with the protein of interest to predict whether the library compound might similarly react. For example, rather than testing every ligand in a large library for interaction with a complex or protein component, only those ligands having a fingerprint similar to other compounds known to have that activity could be tested. (See, e.g., Kauvar et al., 1995, Chem. Biol. 2:107-118; Kauvar, 1995, Affinity fingerprinting, Pharmaceutical Manufacturing International. 8:25-28; and Kauvar, Toxic-Chemical Detection by Pattern Recognition in New Frontiers in Agrochemical Immunoassay, Kurtz, Stanker and Skerritt (eds), 1995, AOAC: Washington, D.C., 305-312).

Kay et al. (1993, Gene 128:59-65) disclosed a method of constructing peptide libraries that encode peptides of totally random sequence that are longer than those of any prior conventional libraries. The libraries disclosed in Kay et al. encode totally synthetic random peptides of greater than about 20 amino acids in length. Such libraries can be advantageously screened to identify complex modulators. (See also U.S. Patent No. 5,498,538 dated March 12, 1996; and PCT Publication No. WO 94/18318 dated August 18, 1994).

A comprehensive review of various types of peptide libraries can be found in Gallop et al., 1994, J. Med. Chem. 37:1233-1251.

In a preferred embodiment, the interaction of the test compound with GPR49 results in an inhibition of GPR49-activity.

According to a preferred embodiment, in step b) the ability of the gamma-secretase to cleave APP is measured. This can be measured as indicated above.

Further, the invention also describes a method for preparing a pharmaceutical composition for the treatment of neurodegenerative diseases, possibly Alzheimer's disease, comprising the following steps:
a) identifying a gamma-secretase modulator and/or beta-secretase modulator, preferably inhibitor, according to the method of the invention, and
b) formulating the gamma-secretase and/or beta-secretase modulator, preferably inhibitor, to a pharmaceutical composition.

With respect to the pharmaceutical composition, all embodiments as indicated above apply also here.

This method may further comprise the step of mixing the identified molecule with a pharmaceutically acceptable carrier as explained above.

The invention also describes a pharmaceutical composition comprising a GPR49-inhibitor as defined above.

Furthermore, the invention also describes a pharmaceutical composition obtainable by the above method for the preparation of a pharmaceutical composition.

The invention also describes the pharmaceutical composition as described above for the treatment of a neurodegenerative disease such as Alzheimer's disease and related neurodegenerative disorders.

The invention also describes a method for treating or preventing a neurodegenerative disease, preferably Alzheimer's disease, comprising administering to a subject in need of such treatment or prevention a therapeutically effective amount of a pharmaceutical composition as described above

With respect to that method all embodiments as described above for the use of the invention also apply.

The invention also relates to the use of a GPR49-inhibitor as used above for the modulation, preferably inhibition of beta-secretase and/or gamma-secretase activity in vitro. For example, it is encompassed within the present invention to modulate, preferably inhibit beta-secretase and/or gamma-secretase activity in cell cultures by the GPR49-interacting molecule. All embodiments with respect to the GPR49-inhibitor as described above also apply to this use of the invention.

The following examples will describe the subject-matter of the invention in more detail.

### Example 1

The TAP-technology, which is more fully described in EP 1 105 508 B1 and in Rigaut, et al., 1999, Nature Biotechnol. 17:1030-1032 respectively, was used and further adapted as described below for protein purification. Proteins were identified using mass spectrometry as described further below.

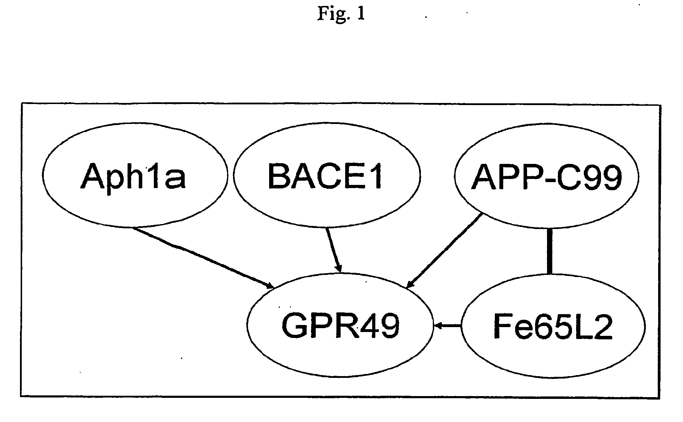

GPR49 was identified as a member of protein complexes with the TAP technology entry points Aph1a, APP-C99, BACE1 and Fe65L2 (Fig. 1)

### Part 1: Construction of TAP-tagged bait

The cDNAs encoding the complete ORF were obtained by RT-PCR. Total RNA was prepared from appropriate cell lines using the RNeasy Mini Kit (Qiagen). Both cDNA synthesis and PCR were performed with the SUPERSCRIPT One-Step RT-PCR for Long templates Kit (Life Technologies) using gene-specific primers. After 35-40 cycles of amplification PCR-products with the expected size were gel-purified with the MinElute PCR Purification Kit (Qiagen) and, if necessary, used for further amplification. Low-abundant RNAs were amplified by nested PCR before gel-purification. Restriction sites for NotI were attached to PCR primers to allow subcloning of amplified cDNAs into the retroviral vectors pIE94-N/C-TAP thereby generating N- or C-terminal fusions with the TAP-tag (Rigaut et al., 1999, Nature Biotechnol. 17:1030-1032). N-terminal tagging was chosen for the following baits/entry points: Presenilin 1, Presenilin 2, Aph-1a, Aph-1b, Pen-2, APP, Tau, Fe65, Calsenilin. C-terminal tagging was chosen for the following baits/entry points: Nicastrin, Aph-1a, Aph-1b, BACE1 D215N, APP, APP695SW, APP-C99, Fe65, Fe65L2, X11beta.

Clones were analyzed by restriction digest, DNA sequencing and by in vitro translation using the TNT T7 Quick Coupled Transcription/Translation System (Promega inc.). The presence of the proteins was proven by Western blotting using the protein A part of the TAP-tag for detection. Briefly, separation of proteins by standard SDS-PAGE was followed by semi-dry transfer onto a nitrocellulose membrane (PROTRAN, Schleicher&Schuell) using the MultiphorII blotting apparatus from Pharmacia Biotech.

The transfer buffer consisted of 48 mM Tris, 39 mM glycine, 10% methanol and 0,0375% sodium dodecylsulfate. After blocking in phosphate-buffered saline (PBS) supplemented with 10% dry milk powder and 0,1% Tween 20 transferred proteins were probed with the Peroxidase-Anti-Peroxidase Soluble Complex (Sigma) diluted in blocking solution. After intensive washing immunoreactive proteins were visualized by enhanced chemiluminescence (ECL; Amersham Pharmacia Biotech).

### Part 2: Preparation of Virus and infection

As a vector, a MoMLV-based recombinant virus was used.

The preparation has been carried out as follows:

### 2.1. Preparation of Virus

293 gp cells were grown to 100% confluency. They were split 1:5 on poly-L-Lysine plates (1:5 diluted poly-L-Lysine [0.01% stock solution, Sigma P-4832] in PBS, left on plates for at least 10 min.). On Day 2, 63 microgram of retroviral Vector DNA together with 13 microgram of DNA of plasmid encoding an appropriate envelope protein were transfected into 293 gp cells (Somia, et al., 1999, Proc. Natl. Acad. Sci. USA 96:12667-12672; Somia, et al. 2000, J. Virol. 74:4420-4424). On Day 3, the medium was replaced with 15 ml DMEM + 10% FBS per 15-cm dish. On Day 4, the medium containing viruses (supernatant) was harvested (at 24 h following medium change after transfection). When a second collection was planned, DMEM 10 % FBS was added to the plates and the plates were incubated for another 24 h. All collections were done as follows: The supernatant was filtered through 0.45 micrometer filter (Corning GmbH, cellulose acetate, 431155). The filter was placed into konical polyallomer centrifuge tubes (Beckman, 358126) that are placed in buckets of a SW 28 rotor (Beckman). The filtered supernatant was ultracentrifuged at 19400 rpm in the SW 28 rotor, for 2 hours at 21 degree Celsius. The supernatant was discarded. The pellet containing viruses was resuspended in a small volume (for example 300 microliter) of Hank's Balanced Salt Solution [Gibco BRL, 14025-092], by pipetting up and down 100-times, using an aerosol-safe tip. The viruses were used for transfection as described below.

### 2.2. Infection

Cells that were infected were plated one day before into one well of a 6-well plate. 4 hours before infection, the old medium on the cells was replaced with fresh medium. Only a minimal volume was added, so that the cells are completely covered (e.g. 700 microliter). During infection, the cells were actively dividing.

A description of the cells and their growth conditions is given further below ("3. Cell lines")

To the concentrated virus, polybrene (Hexadimethrine Bromide; Sigma, H 9268) was added to achieve a final concentration of 8 microgram/ml (this is equivalent to 2.4 microliter of the 1 milligram/ml polybrene stock per 300 microliter of concentrated retrovirus). The virus was incubated in polybrene at room temperature for 1 hour. For infection, the virus/polybrene mixture was added to the cells and incubated at 37 degree Celsius at the appropriate CO₂ concentration for several hours (e.g. over-day or overnight). Following infection, the medium on the infected cells was replaced with fresh medium. The cells were passaged as usual after they became confluent. The cells contain the retrovirus integrated into their chromosomes and stably express the gene of interest.

### 2.3. Cell lines

For expression, SKN-BE2 cells were used. SKN-BE2 cells (American Type Culture Collection-No. CRL-2271) were grown in 95% OptiMEM + 5% iron-supplemented calf serum.

### Part 3: Checking of expression pattern of TAP-tagged proteins

The expression pattern of the TAP-tagged protein was checked by immunoblot analysis and/or by immunofluorescence. Immunofluorescence analysis was either carried out according to No. 1 or to No. 2 depending on the type of the TAP-tagged protein. Immunoblot analysis was carried out according to No. 3.

### 3.1 Protocol for the indirect Immunofluorescence staining of fixed mammalian cells for plasma membrane and ER bound proteins

Cells were grown in FCS media on polylysine coated 8 well chamber slides to 50% confluency. Then fixation of the cells was performed in 4% Paraformaldehyde diluted in Phosphate Buffer Saline (PBS) solution (0.14M Phosphate, 0.1M NaCl pH 7.4). The cells were incubated for 30 minutes at room temperature in 300 microliters per well. Quenching was performed in 0.1M Glycine in PBS for 2x 20 minutes at room temperature. Blocking was performed with 1% Bovine Serum Albumin (BSA) in 0.3% Saponin + PBS for at least 1 hour at room temperature. Incubation of the primary antibodies was performed in the blocking solution overnight at +4°C. The proper dilution of the antibodies was determined in a case to case basis. Cells were washed in PBS containing 0.3% Saponin for 2x 20 minutes at room temperature. Incubation of the secondary antibodies is performed in the blocking solution. Alexa 594 coupled goat anti-rabbit is diluted 1:1000 (Molecular Probes). Alexa 488 coupled goat anti-mouse is diluted 1:1000 (Molecular Probes). DAPI was used to label DNA. If Phalloidin was used to label F-actin, the drug is diluted 1:500 and incubated with the secondary antibodies. Cells were then washed again 2x 20 minutes at room temperature in PBS. The excess of buffer was removed and cells were mounted in a media containing an anti-bleaching agent (Vectashield, Vector Laboratories).

### 3.2 Protocol for the indirect Immunofluorescence staining of fixed mammalian cells for non-plasma membrane bound proteins:

Cells were grown in FCS media on Polylysine coated 8 well chamber slides to 50% confluency. Fixation of the cells was performed in 4% ParaFormAldehyde diluted in Phosphate Buffer Saline (PBS) solution (0.14M Phosphate, 0.1M NaCl pH 7.4) for 30 minutes at Room Temperature (RT), 300 microliters per well. Quenching was performed in 0.1M Glycine in PBS for 2x 20 minutes at roon temperature. Permeabilization of cells was done with 0.5% Triton X-100 in PBS for 10 minutes at room temperature. Blocking was then done in 1% Bovine Serum Albumin (BSA) in 0.3% Saponin + PBS for at least 1 hour at RT (Blocking solution). Incubation of the primary antibodies was performed in the blocking solution, overnight at +4°C. The proper dilution of the antibodies has to be determined in a case to case basis. Cells were washed in PBS containing 0.3% Saponin, for 2x 20 minutes at RT. Incubation of the secondary antibodies was performed in the blocking solution. Alexa 594 coupled goat anti-rabbit is diluted 1:1000 (Molecular Probes), Alexa 488 coupled goat anti-mouse is diluted 1:1000 (Molecular Probes). DAPI was used to label DNA. If Phalloidin is used to label F-actin, the drug is diluted 1:500 and incubated with the secondary antibodies. Cells were washed 2x 20 minutes at RT in PBS.

The excess of buffer was removed and cells were mounted in a media containing an anti-bleaching agent (Vectashield, Vector Laboratories).

### 3.3 Immunoblot analysis

To analyze expression levels of TAP-tagged proteins, a cell pellet (from a 6-well dish) was lyzed in 60 µl DNAse I buffer (5% Glycerol, 100 mM NaCl, 0.8 % NP-40 (IGEPAL), 5 mM magnesium sulfate, 100 µg/ml DNAse I (Roche Diagnostics), 50 mM Tris, pH 7.5, protease inhibitor cocktail) for 15 min on ice. Each sample was split into two aliquots. The first half was centrifuged at 13,000 rpm for 5 min. to yield the NP-40-extractable material in the supernatant; the second half (total material) was carefully triturated. 50 µg each of the NP-40-extractable material and the total material are mixed with DTT-containing sample buffer for 30 min at 50°C on a shaker and separated by SDS polyacrylamide gel electrophoresis on a precast 4-12% Bis-Tris gel (Invitrogen). Proteins were then transferred to nitrocellulose using a semi-dry procedure with a discontinuous buffer system. Briefly, gel and nitrocellulose membrane were stacked between filter papers soaked in either anode buffer (three layers buffer Al (0.3 M Tris-HCl) and three layers buffer A2 (0.03 M Tris-HCl)) or cathode buffer (three layers of 0.03 M Tris-HCl, pH 9.4, 0.1 % SDS, 40 mM □-aminocapronic acid). Electrotransfer of two gels at once was performed at 600 mA for 25 min. Transferred proteins were visualized with Ponceau S solution for one min to control transfer efficiency and then destained in water. The membrane was blocked in 5% non-fat milk powder in TBST (TBS containing 0.05% Tween-20) for 30 min at room temperature. It was subsequently incubated with HRP-coupled PAP antibody (1:5000 diluted in 5% milk/TBST) for 1 h at room temperature, washed three times for 10 min in TBST. The blot membrane was finally soaked in chemiluminescent substrate (ECL, Roche Diagnostics) for 2 min. and either exposed to X-ray film or analyzed on an imaging station.

### Part 4 Purification or protein complexes

Protein complex purification was adapted to the sub-cellular localization of the TAP-tagged protein and was performed as described below.

### 4.1 Lysate preparation for cytoplasmic proteins

About 1 x 10⁹ adherent cells (average) were harvested with a cell scrapper and washed 3 times in ice-cold PBS (3 min, 550g). Collected cells were frozen in liquid nitrogen or immediately processed further. For cell lysis, the cell pellet was resuspended in 10 ml of CZ lysis buffer (50 mM Tris-Cl, pH 7.4; 5 % Glycerol; 0,2 % IGEPAL; 1.5 mM MgCl₂; 100 mM NaCl; 25 mM NaF; 1 mM Na₃VO₄; 1 mM DTT; containing 1 tablet of EDTA-free Protease inhibitor cocktail (Complete^{™}, Roche) per 25 ml of buffer) and homogenized by 10 strokes of a tight-fitted pestle in a dounce homogenizer. The lysate was incubated for 30 min on ice and spun for 10 min at 20,000g. The supernatant was subjected to an additional ultracentrifugation step for 1 h at 100,000g. The supernatant was recovered and rapidly frozen in liquid nitrogen or immediately processed further.

### 4.2 Lysate preparation for membrane proteins

About 1 x 10⁹ adherent cells (average) were harvested with a cell scrapper and washed 3 times in ice-cold PBS (3 min, 550g). Collected cells were frozen in liquid nitrogen or immediately processed further. For cell lysis, the cell pellet was resuspended in 10 ml of Membrane-Lysis buffer (50 mM Tris, pH 7.4; 7.5 % Glycerol; 1 mM EDTA; 150 mM NaCl; 25 mM NaF; 1 mM Na₃VO₄; 1 mM DTT; containing 1 tablet of EDTA-free Protease inhibitor cocktail (Complete^{™}, Roche) per 25 ml of buffer) and homogenized by 10 strokes of a tight-fitted pestle in a dounce homogenizer. The lysate was spun for 10 min at 750g, the supernatant was recovered and subjected to an ultracentrifugation step for 1 h at 100,000g. The membrane pellet was resuspended in 7,5 ml of Membrane-Lysis buffer containing 0.8% n-Dodecyl-D-maltoside and incubated for 1 h at 4°C with constant agitation. The sample was subjected to another ultracentifugation step for 1h at 100,000g and the solubilized material was quickly frozen in liquid nitrogen or immediately processed further.

### 4.3 Lysate preparation for nuclear proteins

About 1 x 10⁹ adherent cells (average) were harvested with a cell scrapper and washed 3 times in ice-cold PBS (3 min, 550g). Collected cells were frozen in liquid nitrogen or immediately processed further. For cell lysis, the cell pellet was resuspended in 10 ml of Hypotonic-Lysis buffer (10 mM Tris, pH 7.4; 1.5 mM MgCl₂; 10 mM KCl; 25 mM NaF; 1 mM Na₃VO₄; 1 mM DTT; containing 1 tablet of EDTA-free Protease inhibitor cocktail (Complete^{™}, Roche) per 25 ml of buffer) and homogenized by 10 strokes of a tight-fitted pestle in a dounce homogenizer. The lysate was spun for 10 min at 2,000g and the resulting supernatant (S1) saved on ice. The nuclear pellet (P1) was resuspended in 5 ml Nuclear-Lysis buffer (50 mM Tris, pH 7.4; 1.5 mM MgCl₂; 20 % Glycerol; 420 mM NaCI; 25 mM NaF; 1 mM Na₃VO₄; 1 mM DTT; containing 1 tablet of EDTA-free Protease inhibitor cocktail (Complete^{™}, Roche) per 25 ml of buffer) and incubated for 30 min on ice. The sample was combined with S1, further diluted with 7 ml of Dilution buffer (110 mM Tris, pH 7.4; 0.7 % NP40; 1.5 mM MgCl₂; 25 mM NaF; 1 mM Na₃VO₄; 1 mM DTT), incubated on ice for 10 min and centrifuged at 100,000g for 1h. The final supernatant (S2) was frozen quickly in liquid nitrogen.

### 4.4 Tandem Affinity Purification

The frozen lysate was quickly thawed in a 37°C water bath, and spun for 20 min at 100,000g. The supernatant was recovered and incubated with 0.2 ml of settled rabbit IgG-Agarose beads (Sigma) for 2 h with constant agitation at 4°C. Immobilized protein complexes were washed with 10 ml of CZ lysis buffer (containing 1 Complete^{™} tablet (Roche) per 50 ml of buffer) and further washed with 5 ml of TEV cleavage buffer (10 mM Tris, pH 7.4; 100 mM NaCl; 0.1 % IGEPAL; 0.5 mM EDTA; 1 mM DTT). Protein-complexes were eluted by incubation with 5µl of TEV protease (GibcoBRL, Cat.No. 10127-017) for 1 h at 16°C in 150 µl TEV cleavage buffer. The eluate was recovered and combined with 0.2 ml settled Calmodulin affinity beads (Stratagene) in 0.2 ml CBP binding buffer (10 mM Tris, pH 7.4; 100 mM NaCl; 0,1 % IGEPAL; 2mM MgAc; 2mM Imidazole; 1mM DTT; 4 mM CaCl₂) followed by 1 h incubation at 4°C with constant agitation. Immobilized protein complexes were washed with 10 ml of CBP wash buffer (10 mM Tris, pH 7.4; 100 mM NaCl; 0,1 % IGEPAL; 1mM MgAc; 1mM Imidazole; 1mM DTT; 2 mM CaCl₂) and eluted by addition of 600 µl CBP elution buffer (10 mM Tris, pH 8.0; 5 mM EGTA) for 5 min at 37°C. The eluate was recovered in a siliconzed tube and lyophilized. The remaining Calmodulin resin was boiled for 5 min in 50 µl 4x Laemmli sample buffer. The sample buffer was isolated, combined with the lyophilised fraction and loaded on a NuPAGE gradient gel (Invitrogen, 4-12%, 1.5 mm, 10 well).

### Part 5 Protein Identification by Mass Spectrometry

### 5.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 min). Reduced and alkylated proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 5.2 Sample preparation prior to analysis by mass spectrometry

Gel plugs were extracted twice with 20 µl 1% TFA and pooled with acidified digest supernatants. Samples were dried in a a vaccum centrifuge and resuspended in 13 µl 1% TFA.

### 5.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass or QSTAR Pulsar, Sciex) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

| | | |
|---|---|---|
| Time (min) | % solvent A | % solvent B |
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

Peptides eluting off the LC system were partially sequenced within the mass spectrometer.

### 5.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999, Electrophoresis 20:3551-3567). Search criteria varied depending on which mass spectrometer was used for the analysis.

### Example 2: Effect of siRNA-mediated knock-down of GPR49 on Aβ1-42 levels

### Result:

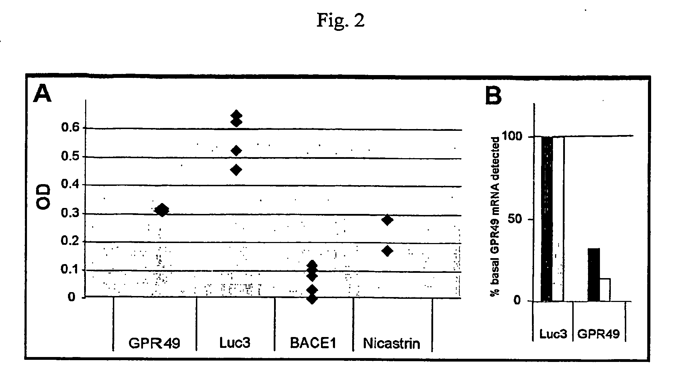

We noticed that like siRNAs directed against the known effectors of APP processing, BACE1 and nicastrin, the siRNA targeting GPR49 caused significant attenuation of Aβ1-42 secretion, whereas the Luc3 siRNA had no effect (Figure 2A) - demonstrating that GPR49 plays a functional role in regulating the processing/secretion of APP.

We confirmed that the GPR49 siRNA did indeed interfere with expression of GPR49 (Figure 2B).

A RNAi gene expression perturbation strategy was employed for functional validation of GPR49 as an effector of APP processing: An siRNA directed against GPR49 or siRNAs directed against known effectors of APP processing, BACE1 or nicastrin, or against unrelated Luc3 was transfected into SK-N-BE2 neuroblastoma cells expressing human APP695. The siRNA for human GPR49 were synthesized by Dharmacon Research Inc.

The siRNA sequence used for GPR49 was: AACAGCAGTATGGACGACCTT.

Transfection of SK-N-BE2 cells was performed using LipofectAMINE 2000 (Invitrogen) following the manufacturer's instructions. Briefly, the cells were seeded at a density of 1.0 x 10⁴ cells in a final volume of 85 µl per 96-well 12-16 hrs prior to transfection. 25 nM of siRNAs were mixed with 8 µl Opti-MEM buffer (Gibco) and 60 ng carrier DNA, and the mixture was incubated for 20 minutes at room temperature before addition to the cells. 16 and 48 hrs post-transfection medium was replaced with 100 µl or 200 µl growth medium with or without serum, respectively. 72 hrs post-transfection 100 µl supernatants were harvested for Aβ1-42 ELISA (Innogenetics). The assay was performed following the manufacturer's instructions.

Knockdown efficiency of selected siRNAs was assessed by quantitative RT-PCR. Briefly, 5x 10°5 SKNBE2 cells were plated per 6-well and transfected with 25 nM siRNA the following day. 36 h after transfection, cells were harvested and total RNA was prepared and reverse-transcribed using standard procedures. Equal amounts of cDNAs and GPR49-specific primers were utilized for determination of relative expression levels of GPR49 following manufacturer's instructions. All values were normalized to a human reference RNA (Stratagene).

### Example 3: Determination of GPR49 activity

Signal transduction cascades triggered by GPR49 are currently unknown. Other family members, however, human glycohormone receptors as well as the phylogenetically distant putative LGR ortholog in nematodes (Kudo M, Chen T, Nakabayashi K, Hsu SY, Hsueh AJ. (2000) The nematode leucine-rich repeat-containing, G protein-coupled receptor (LGR) protein homologous to vertebrate gonadotropin and thyrotropin receptors is constitutively active in mammalian cells. Mol Endocrinol. 14(2):272-84), have been shown to signal through adenylate cyclase-dependent mechanisms. The latter, when heterologously expressed in mammalian cells causes constitutive increases in cellular cyclic adenosine monophosphate (cAMP) levels.

To confirm that GPR49 does couple to the cAMP pathway several assays available in the public domain can be used. For instance, Bresnick et al. (Bresnick JN, Skynner HA, Chapman KL, Jack AD, Zamiara E, Negulescu P, Beaumont K, Patel S, McAllister G (2003) Identification of signal transduction pathways used by orphan g protein-coupled receptors. Assay Drug Dev Technol. 1(2):239-49.) have used beta-lactamase reporter constructs to identify signal transduction pathways used by orphan GPRCs.

GPR49 modulators are then identified based on their ability to trigger cellular elevations in cAMP that are observed in GPR49-expressing but not in GPR49-deficient cells. Direct measurements of cAMP can, for example, be done in a high-troughput format using cAMP-response element (CRE)-luciferase reporter cell lines (Gabriel D, Vernier M, Pfeifer MJ, Dasen B, Tenaillon L, Bouhelal R (2003) High throughput screening technologies for direct cyclic AMP measurement. Assay Drug Dev Technol. 1(2):291-303). Other methods for determination of cAMP elevations or cAMP-dependent signaling are apparent to a person skilled in the art. For a review of precedents for identification of small molecule modulators of previously orphan GPCRs, see (Howard AD, McAllister G, Feighner SD, Liu Q, Nargund RP, Van der Ploeg LH, Patchett AA (2001) Orphan G-protein-coupled receptors and natural ligand discovery. Trends Pharmacol Sci. 22(3):132-40).

### Example 4: Modulation of Aβ1-42 generation/secretion by GPR49 modulators

SKNBE2 cells (or another suitable cell line) stably over-expressing human APP695 (ShNBE2/APP695) or a suitable mutant with enhanced beta-/gamma-secretase cleavage kinetics are plated in growth medium and serum-starved for 4 h the next morning. An GPR49 modulator, preferably inhibitor, diluted in serum-free medium, is then added and incubated for suitable periods of time. Cell supernatants are collected and levels of Aβ1-42 determined by ELISA (Innotest β-amyloid (1-42) from INNOGENETICS N.V., Belgium).

The invention is described in more detail in the following figures:
- Figure 1:: Schematic representation of TAP entry points (white) that GPR49 was found to interact with.
- Figure 2:: siRNA-mediated knock-down of GPR49 expression attenuates generation/secretion of Aβ1-42.
Fig. 2A: siRNAs directed against BACE1, nicastrin, GPR49 or Luc3 were transfected into SK-N-BE2 neuroblastoma cells over-expressing APP695. 48h after transfection growth medium was removed and cells were incubated over night in serum-free medium. Supernatants were collected and levels of Aβ1-42 determined by ELISA (Innotest β-amyloid (1-42) from INNOGENETICS N.V., Belgium). At least three independent experiments were performed in duplicate. A representative example is shown.
Fig. 2B: An siRNA directed against GPR49, but not one directed against unrelated Luc3 specifically reduces GPR49 mRNA as assessed by quantitative RT-PCR analysis. Two bars shown for each siRNA represent two independent experiments.
- Figure 3:: Amino acid sequence of human GPR (LGR5; leucine-rich repeat-containing G protein-coupled receptor 5), depicted in the one-letter-code.
- Figure 4:: Multiple sequence alignment of human LGR4, LGR5/GPR49 and LGR6

## Claims

1. Use of a GPR49 inhibitor selected from the group consisting of antibodies, antisense oligonucleotides, siRNA, and ribozymes for the preparation of a pharmaceutical composition for the treatment of a neurodegenerative disease.

2. The use of claim 1, wherein GPR49 is part of an intracellular protein complex.

3. The use of any of claims 1 or 2, wherein the inhibitor modulates the activity of gamma-secretase and/or beta-secretase.

4. The use of any of claims 1 to 3, wherein the neurodegenerative disease is Alzheimer's disease.

5. A method for identifying a gamma-secretase and/or a beta-secretase modulator, comprising the following steps:
a. identifying of a GPR49-interacting molecule by determining whether a given test compound is a GPR49-interacting molecule,
b. determining whether the GPR49-interacting molecule of step a) is capable of modulating gamma-secretase and/or beta-secretase activity.

6. The method of claim 5, wherein in step a) the test compound is brought into contact with GPR49 and the interaction of GPR49 with the test compound is determined.

7. The method of claim 6, wherein the interaction of the test compound with GPR49 results in an inhibition of GPR49 activity.

8. The method of any of claims 5 to 7, wherein in step b) the ability of the gamma-secretase and/or the beta-secrease to cleave APP is measured, preferably wherein the ability to produce Abeta 42 is measured.

9. Use of a GPR49-inhibitor for the inhibition of beta-secretase and/or gamma-secretase activity in vitro, wherein the inhibitor is a molecule selected from the group consisting of antibodies, antisense oligonucleotides, siRNA, and ribozymes.

## Patentansprüche

1. Verwendung eines GPR49-Inhibitors ausgewählt aus der Gruppe bestehend aus Antikörpern, Antisense-Oligonukleotiden, siRNA und Ribozymen zur Herstellung einer pharmazeutischen Zusammensetzung zur Behandlung einer neurodegenerativen Erkrankung.

2. Verwendung nach Anspruch 1, wobei GPR49 Teil eines intrazellulären Proteinkomplexes ist.

3. Verwendung nach irgendeinem der Ansprüche 1 oder 2, wobei der Inhibitor die Aktivität der Gamma-Sekretase und/oder Beta-Sekretase moduliert.

4. Verwendung nach irgendeinem der Ansprüche 1 bis 3, wobei die neurodegenerative Erkrankung die Alzheimer'sche Erkrankung ist.

5. Verfahren zum Identifizieren eines Gamma-Sekretase- und/oder eines Beta-Sekretase-Modulators, umfassend die folgenden Schritte:
a. Identifizieren eines mit GPR49 interagierenden Moleküls durch Bestimmen, ob eine bestimmte Testverbindung ein mit GPR49 interagierendes Molekül ist,
b. Bestimmen, ob das mit GPR49 interagierende Molekül von Schritt a) in der Lage ist, die Gamma-Sekretase- und/oder Beta-Sekretaseaktivität zu modulieren.

6. Verfahren nach Anspruch 5, wobei in Schritt a) die Testverbindung in Kontakt mit GPR49 gebracht wird und die Interaktion von GPR49 mit der Testverbindung bestimmt wird.

7. Verfahren nach Anspruch 6, wobei die Interaktion der Testverbindung mit GPR49 zu einer Inhibition der GPR49-Aktivität führt.

8. Verfahren nach irgendeinem der Ansprüche 5 bis 7, wobei in Schritt b) die Fähigkeit der Gamma-Sekretase und/oder der Beta-Sekretase APP zu spalten gemessen wird, vorzugsweise wobei die Fähigkeit zum Herstellen von Abeta 42 gemessen wird.

9. Verwendung eines GPR49-Inhibitors zur Inhibition der Beta-Sekretase- und/oder Gamma-Sekretaseaktivität in vitro, wobei der Inhibitor ein Molekül ist ausgewählt aus der Gruppe bestehend aus Antikörpern, Antisense-Oligonukleotiden, siRNA und Ribozymen.

## Revendications

1. Utilisation d'un inhibiteur de GPR49 choisi dans le groupe composé des anticorps, oligonucléotides antisens, ARNi et ribozymes pour la préparation d'une composition pharamaceutique pour le traitement d'une maladie neurodégénérative

2. L'utilisation selon la revendication 1, dans laquelle GPR49 fait partie d'un complexe protéinique intracellulaire.

3. L'utilisation selon la revendication 1 ou 2, dans laquelle l'inhibiteur module l'activité de la gamma-secrétase et/ou de la béta-secrétase.

4. L'utilisation selon une quelconque des revendications 1 à 3, dans laquelle la maladie neurodégénérative est la maladie d'Alzheimer.

5. Un procédé pour identifier un modulateur de gamma-secrétase et/ou de la beta-secrétase, comprenant les étapes suivantes:
a) identification d'une molécule interagissant avec GPR49 en déterminant si un composé testé donné est une molécule interagissant avec GPR49,
b) détermination de la capacité de la molécule interagissant avec GPR49 de l'étape a), à moduler l'activité de la gamma-secrétase et/ou de la béta-secrétase.

6. Le procédé selon la revendication 5, dans lequel dans l'étape a) le composé testé est mis en contact avec GPR49 et l'interaction de GPR49 avec le composé testé est déterminée.

7. Le procédé selon la revendication 6, dans lequel l'interaction du composé testé avec GPR49 conduit à une inhibition de l'activité de GPR49.

8. Le procédé selon une quelconque des revendications 5 à 7, dans lequel dans l'étape b) la capacité de la gamma-secrétase et/ou de la béta-secrétase à cliver l'app est mesurée, de préférence dans lequel la capacité à produire Abeta 42 est mesurée.

9. Utilisation d'un inhibiteur de GPR49 pour l'inhibition in vitro de l'activité gamma-secrétase et/ou béta-secrétase, dans laquelle l'inhibiteur est une molécule choisie dans le groupe comosé des anticorps, oligonucléotides antisens, ARNi et ribozymes.


### SEQUENCE LISTING

<110> Cellzome AG
<120> Treatment of Neurodegenerative Diseases by the use of GPR49
<130> CEL63172PC
<140> PCT/EP2004/013539
   <141> 2004-11-29
<160> 4
<170> PatentIn version 3.1
<210> 1
   <211> 21
   <212> DNA
   <213> Artificial sequence
<220>
   <223> designed siRNA
<400> 1
   aacagcagta tggacgacct t 21
<210> 2
   <211> 907
   <212> PRT
   <213> Homo sapiens
<400> 2
   Met Asp Thr Ser Arg Leu Gly Val Leu Leu Ser Leu Pro Val Leu Leu
   1               5                   10                  15
   Gln Leu Ala Thr Gly Gly Ser Ser Pro Arg Ser Gly Val Leu Leu Arg
               20                  25                  30
   Gly Cys Pro Thr His Cys His Cys Glu Pro Asp Gly Arg Met Leu Leu
   35 40 45
   Arg Val Asp Cys Ser Asp Leu Gly Leu Ser Glu Leu Pro Ser Asn Leu
   50 55 60
   Ser Val Phe Thr Ser Tyr Leu Asp Leu Ser Met Asn Asn Ile Ser Gln
   65 70 75 80
   Leu Leu Pro Asn Pro Leu Pro Ser Leu Arg Phe Leu Glu Glu Leu Arg
   85 90 95
   Leu Ala Gly Asn Ala Leu Thr Tyr Ile Pro Lys Gly Ala Phe Thr Gly
   100 105 110
   Leu Tyr Ser Leu Lys Val Leu Met Leu Gln Asn Asn Gln Leu Arg His
   115 120 125
   Val Pro Thr Glu Ala Leu Gln Asn Leu Arg Ser Leu Gln Ser Leu Arg
   130 135 140
   Leu Asp Ala Asn His Ile Ser Tyr Val Pro Pro Ser Cys Phe Ser Gly
   145 150 155 160
   Leu His Ser Leu Arg His Leu Trp Leu Asp Asp Asn Ala Leu Thr Glu
   165 170 175
   Ile Pro Val Gln Ala Phe Arg Ser Leu Ser Ala Leu Gln Ala Met Thr
   180 185 190
   Leu Ala Leu Asn Lys Ile His His Ile Pro Asp Tyr Ala Phe Gly Asn
   195 200 205
   Leu Ser Ser Leu Val Val Leu His Leu His Asn Asn Arg Ile His Ser
   210 215 220
   Leu Gly Lys Lys Cys Phe Asp Gly Leu His Ser Leu Glu Thr Leu Asp
   225 230 235 240
   Leu Asn Tyr Asn Asn Leu Asp Glu Phe Pro Thr Ala Ile Arg Thr Leu
   245 250 255
   Ser Asn Leu Lys Glu Leu Gly Phe His Ser Asn Asn Ile Arg Ser Ile
   260 265 270
   Pro Glu Lys Ala Phe Val Gly Asn Pro Ser Leu Ile Thr Ile His Phe
   275 280 285
   Tyr Asp Asn Pro Ile Gln Phe Val Gly Arg Ser Ala Phe Gln His Leu
   290 295 300
   Pro Glu Leu Arg Thr Leu Thr Leu Asn Gly Ala Ser Gln Ile Thr Glu
   305 310 315 320
   Phe Pro Asp Leu Thr Gly Thr Ala Asn Leu Glu Ser Leu Thr Leu Thr
   325 330 335
   Gly Ala Gln Ile Ser Ser Leu Pro Gln Thr Val Cys Asn Gln Leu Pro
   340 345 350
   Asn Leu Gln Val Leu Asp Leu Ser Tyr Asn Leu Leu Glu Asp Leu Pro
   355 360 365
   Ser Phe Ser Val Cys Gln Lys Leu Gln Lys Ile Asp Leu Arg His Asn
   370 375 380
   Glu Ile Tyr Glu Ile Lys Val Asp Thr Phe Gln Gln Leu Leu Ser Leu
   385 390 395 400
   Arg Ser Leu Asn Leu Ala Trp Asn Lys Ile Ala Ile Ile His Pro Asn
   405 410 415
   Ala Phe Ser Thr Leu Pro Ser Leu Ile Lys Leu Asp Leu Ser Ser Asn
   420 425 430
   Leu Leu Ser Ser Phe Pro Ile Thr Gly Leu His Gly Leu Thr His Leu
   435 440 445
   Lys Leu Thr Gly Asn His Ala Leu Gln Ser Leu Ile Ser Ser Glu Asn
   450 455 460
   Phe Pro Glu Leu Lys Val Ile Glu Met Pro Tyr Ala Tyr Gln Cys Cys
   465 470 475 480
   Ala Phe Gly Val Cys Glu Asn Ala Tyr Lys Ile Ser Asn Gln Trp Asn
   485 490 495
   Lys Gly Asp Asn Ser Ser Met Asp Asp Leu His Lys Lys Asp Ala Gly
   500 505 510
   Met Phe Gln Ala Gln Asp Glu Arg Asp Leu Glu Asp Phe Leu Leu Asp
   515 520 525
   Phe Glu Glu Asp Leu Lys Ala Leu His Ser Val Gln Cys Ser Pro Ser
   530 535 540
   Pro Gly Pro Phe Lys Pro Cys Glu His Leu Leu Asp Gly Trp Leu Ile
   545 550 555 560
   Arg Ile Gly Val Trp Thr Ile Ala Val Leu Ala Leu Thr Cys Asn Ala
   565 570 575
   Leu Val Thr Ser Thr Val Phe Arg Ser Pro Leu Tyr Ile Ser Pro Ile
               580                 585                 590
   Lys Leu Leu Ile Gly Val Ile Ala Ala Val Asn Met Leu Thr Gly Val
           595                 600                 605
   Ser Ser Ala Val Leu Ala Gly Val Asp Ala Phe Thr Phe Gly Ser Phe
       610                 615                 620
   Ala Arg His Gly Ala Trp Trp Glu Asn Gly Val Gly Cys His Val Ile
   625                 630                 635                 640
   Gly Phe Leu Ser Ile Phe Ala Ser Glu Ser Ser Val Phe Leu Leu Thr
                   645                 650                 655
   Leu Ala Ala Leu Glu Arg Gly Phe Ser Val Lys Tyr Ser Ala Lys Phe
               660                 665                 670
   Glu Thr Lys Ala Pro Phe Ser Ser Leu Lys Val Ile Ile Leu Leu Cys
           675                 680                 685
   Ala Leu Leu Ala Leu Thr Met Ala Ala Val Pro Leu Leu Gly Gly Ser
       690                 695                 700
   Lys Tyr Gly Ala Ser Pro Leu Cys Leu Pro Leu Pro Phe Gly Glu Pro
   705                 710                 715                 720
   Ser Thr Met Gly Tyr Met Val Ala Leu Ile Leu Leu Asn Ser Leu Cys
                   725                 730                 735
   Phe Leu Met Met Thr Ile Ala Tyr Thr Lys Leu Tyr Cys Asn Leu Asp
               740                 745                 750
   Lys Gly Asp Leu Glu Asn Ile Trp Asp Cys Ser Met Val Lys His Ile
           755                 760                 765
   Ala Leu Leu Leu Phe Thr Asn Cys Ile Leu Asn Cys Pro Val Ala Phe
       770                 775                 780
   Leu Ser Phe Ser Ser Leu Ile Asn Leu Thr Phe Ile Ser Pro Glu Val
   785                 790                 795                 800
   Ile Lys Phe Ile Leu Leu Val Val Val Pro Leu Pro Ala Cys Leu Asn
                   805                 810                 815
   Pro Leu Leu Tyr Ile Leu Phe Asn Pro His Phe Lys Glu Asp Leu Val
               820                 825                 830
   Ser Leu Arg Lys Gln Thr Tyr Val Trp Thr Arg Ser Lys His Pro Ser
           835                 840                 845
   Leu Met Ser Ile Asn Ser Asp Asp Val Glu Lys Gln Ser Cys Asp Ser
   850 855 860
   Thr Gln Ala Leu Val Thr Phe Thr Ser Ser Ser Ile Thr Tyr Asp Leu
   865 870 875 880
   Pro Pro Ser Ser Val Pro Ser Pro Ala Tyr Pro Val Thr Glu Ser Cys
   885 890 895
   His Leu Ser Ser Val Ala Phe Val Pro Cys Leu
   900 905
<210> 3
   <211> 828
   <212> PRT
   <213> Homo sapiens
<400> 3
   Met Arg Leu Glu Gly Glu Gly Arg Ser Ala Arg Ala Gly Gln Asn Leu
   1 5 10 15
   Ser Arg Ala Gly Ser Ala Arg Arg Gly Ala Pro Arg Asp Leu Ser Met
   20 25 30
   Asn Asn Leu Thr Glu Leu Gln Pro Gly Leu Phe His His Leu Arg Phe
   35 40 45
   Leu Glu Glu Leu Arg Leu Ser Gly Asn His Leu Ser His Ile Pro Gly
   50 55 60
   Gln Ala Phe Ser Gly Leu Tyr Ser Leu Lys Ile Leu Met Leu Gln Asn
   65 70 75 80
   Asn Gln Leu Gly Gly Ile Pro Ala Glu Ala Leu Trp Glu Leu Pro Ser
   85 90 95
   Leu Gln Ser Leu Asp Leu Asn Tyr Asn Lys Leu Gln Glu Phe Pro Val
   100 105 110
   Ala Ile Arg Thr Leu Gly Arg Leu Gln Glu Leu Gly Phe His Asn Asn
   115 120 125
   Asn Ile Lys Ala Ile Pro Glu Lys Ala Phe Met Gly Asn Pro Leu Leu
   130 135 140
   Gln Thr Ile His Phe Tyr Asp Asn Pro Ile Gln Phe Val Gly Arg Ser
   145 150 155 160
   Ala Phe Gln Tyr Leu Pro Lys Leu His Thr Leu Ser Leu Asn Gly Ala
   165 170 175
   Met Asp Ile Gln Glu Phe Pro Asp Leu Lys Gly Thr Thr Ser Leu Glu
   180 185 190
   Ile Leu Thr Leu Thr Arg Ala Gly Ile Arg Leu Leu Pro Ser Gly Met
   195 200 205
   Cys Gln Gln Leu Pro Arg Leu Arg Val Leu Glu Leu Ser His Asn Gln
   210 215 220
   Ile Glu Glu Leu Pro Ser Leu His Arg Cys Gln Lys Leu Glu Glu Ile
   225 230 235 240
   Gly Leu Gln His Asn Arg Ile Trp Glu Ile Gly Ala Asp Thr Phe Ser
   245 250 255
   Gln Leu Ser Ser Leu Gln Ala Leu Asp Leu Ser Trp Asn Ala Ile Arg
   260 265 270
   Ser Ile His Pro Glu Ala Phe Ser Thr Leu His Ser Leu Val Lys Leu
   275 280 285
   Asp Leu Thr Asp Asn Gln Leu Thr Thr Leu Pro Leu Ala Gly Leu Gly
   290 295 300
   Gly Leu Met His Leu Lys Leu Lys Gly Asn Leu Ala Leu Ser Gln Ala
   305 310 315 320
   Phe Ser Lys Asp Ser Phe Pro Lys Leu Arg Ile Leu Glu Val Pro Tyr
   325 330 335
   Ala Tyr Gln Cys Cys Pro Tyr Gly Met Cys Ala Ser Phe Phe Lys Ala
   340 345 350
   Ser Gly Gln Trp Glu Ala Glu Asp Leu His Leu Asp Asp Glu Glu Ser
   355 360 365
   Ser Lys Arg Pro Leu Gly Leu Leu Ala Arg Gln Ala Glu Asn His Tyr
   370 375 380
   Asp Gln Asp Leu Asp Glu Leu Gln Leu Glu Met Glu Asp Ser Lys Pro
   385 390 395 400
   His Pro Ser Val Gln Cys Ser Pro Thr Pro Gly Pro Phe Lys Pro Cys
   405 410 415
   Glu Tyr Leu Phe Glu Ser Trp Gly Ile Arg Leu Ala Val Trp Ala Ile
   420 425 430
   Val Leu Leu Ser Val Leu Cys Asn Gly Leu Val Leu Leu Thr Val Phe
   435 440 445
   Ala Gly Gly Pro Val Pro Leu Pro Pro Val Lys Phe Val Val Gly Ala
   450 455 460
   Ile Ala Gly Ala Asn Thr Leu Thr Gly Ile Ser Cys Gly Leu Leu Ala
   465 470 475 480
   Ser Val Asp Ala Leu Thr Phe Gly Gln Phe Ser Glu Tyr Gly Ala Arg
   485 490 495
   Trp Glu Thr Gly Leu Gly Cys Arg Ala Thr Gly Phe Leu Ala Val Leu
   500 505 510
   Gly Ser Glu Ala Ser Val Leu Leu Leu Thr Leu Ala Ala Val Gln Cys
   515 520 525
   Ser Val Ser Val Ser Cys Val Arg Ala Tyr Gly Lys Ser Pro Ser Leu
   530 535 540
   Gly Ser Val Arg Ala Gly Val Leu Gly Cys Leu Ala Leu Ala Gly Leu
   545 550 555 560
   Ala Ala Ala Leu Pro Leu Ala Ser Val Gly Glu Tyr Gly Ala Ser Pro
   565 570 575
   Leu Cys Leu Pro Tyr Ala Pro Pro Glu Gly Gln Pro Ala Ala Leu Gly
   580 585 590
   Phe Thr Val Ala Leu Val Met Met Asn Ser Phe Cys Phe Leu Val Val
   595 600 605
   Ala Gly Ala Tyr Ile Lys Leu Tyr Cys Asp Leu Pro Arg Gly Asp Phe
   610 615 620
   Glu Ala Val Trp Asp Cys Ala Met Val Arg His Val Ala Trp Leu Ile
   625 630 635 640
   Phe Ala Asp Gly Leu Leu Tyr Cys Pro Val Ala Phe Leu Ser Phe Ala
   645 650 655
   Ser Met Leu Gly Leu Phe Pro Val Thr Pro Glu Ala Val Lys Ser Val
   660 665 670
   Leu Leu Val Val Leu Pro Leu Pro Ala Cys Leu Asn Pro Leu Leu Tyr
   675 680 685
   Leu Leu Phe Asn Pro His Phe Arg Asp Asp Leu Arg Arg Leu Arg Pro
   690 695 700
   Arg Ala Gly Asp Ser Gly Pro Leu Ala Tyr Ala Ala Ala Gly Glu Leu
   705 710 715 720
   Glu Lys Ser Ser Cys Asp Ser Thr Gln Ala Leu Val Ala Phe Ser Asp
   725 730 735
   Val Asp Leu Ile Leu Glu Ala Ser Glu Ala Gly Arg Pro Pro Gly Leu
   740 745 750
   Glu Thr Tyr Gly Phe Pro Ser Val Thr Leu Ile Ser Cys Gln Gln Pro
   755 760 765
   Gly Ala Pro Arg Leu Glu Gly Ser His Cys Val Glu Pro Glu Gly Asn
   770 775 780
   His Phe Gly Asn Pro Gln Pro Ser Met Asp Gly Glu Leu Leu Leu Arg
   785 790 795 800
   Ala Glu Gly Ser Thr Pro Ala Gly Gly Gly Leu Ser Gly Gly Gly Gly
   805 810 815
   Phe Gln Pro Ser Gly Leu Ala Phe Ala Ser His Val
   820 825
<210> 4
   <211> 955
   <212> PRT
   <213> Homo sapiens
<400> 4
   Met Pro Gly Pro Leu Gly Leu Leu Cys Phe Leu Ala Leu Gly Leu Leu
   1 5 10 15
   Gly Ser Ala Gly Pro Ser Gly Ala Ala Pro Pro Leu Cys Ala Ala Pro
   20 25 30
   Cys Ser Cys Asp Gly Asp Arg Arg Val Asp Cys Ser Gly Lys Gly Leu
   35 40 45
   Thr Ala Val Pro Glu Gly Leu Ser Ala Phe Thr Gln Ala Leu Asp Ile
   50 55 60
   Ser Met Asn Asn Ile Thr Gln Leu Pro Glu Asp Ala Phe Lys Asn Phe
   65 70 75 80
   Pro Phe Leu Glu Glu Leu Gln Leu Ala Gly Asn Asp Leu Ser Phe Ile
   85 90 95
   His Pro Lys Ala Leu Ser Gly Leu Lys Glu Leu Lys Val Leu Thr Leu
               100                 105                 110
   Gln Asn Asn Gln Leu Lys Thr Val Pro Ser Glu Ala Ile Arg Gly Leu
           115                 120                 125
   Ser Ala Leu Gln Ser Leu Arg Leu Asp Ala Asn His Ile Thr Ser Val
       130                 135                 140
   Pro Glu Asp Ser Phe Glu Gly Leu Val Gln Leu Arg His Leu Trp Leu
   145                 150                 155                 160
   Asp Asp Asn Ser Leu Thr Glu Val Pro Val His Pro Leu Ser Asn Leu
                   165                 170                 175
   Pro Thr Leu Gln Ala Leu Thr Leu Ala Leu Asn Lys Ile Ser Ser Ile
               180                 185                 190
   Pro Asp Phe Ala Phe Thr Asn Leu Ser Ser Leu Val Val Leu His Leu
           195                 200                 205
   His Asn Asn Lys Ile Arg Ser Leu Ser Gln His Cys Phe Asp Gly Leu
       210                 215                 220
   Asp Asn Leu Glu Thr Leu Asp Leu Asn Tyr Asn Asn Leu Gly Glu Phe
   225                 230                 235                 240
   Pro Gln Ala Ile Lys Ala Leu Pro Ser Leu Lys Glu Leu Gly Phe His
                   245                 250                 255
   Ser Asn Ser Ile Ser Val Ile Pro Asp Gly Ala Phe Asp Gly Asn Pro
               260                 265                 270
   Leu Leu Arg Thr Ile His Leu Tyr Asp Asn Pro Leu Ser Phe Val Gly
           275                 280                 285
   Asn Ser Ala Phe His Asn Leu Ser Asp Leu His Ser Leu Val Ile Arg
       290                 295                 300
   Gly Ala Ser Met Val Gln Gln Phe Pro Asn Leu Thr Gly Thr Val His
   305                 310                 315                 320
   Leu Glu Ser Leu Thr Leu Thr Gly Thr Lys Ile Ser Ser Ile Pro Asn
                   325                 330                 335
   Asn Leu Cys Gln Glu Gln Lys Met Leu Arg Thr Leu Asp Leu Ser Tyr
               340                 345                 350
   Asn Asn Ile Arg Asp Leu Pro Ser Phe Asn Gly Cys His Ala Leu Glu
           355                 360                 365
   Glu Ile Ser Leu Gln Arg Asn Gln Ile Tyr Gln Ile Lys Glu Gly Thr
       370                 375                 380
   Phe Gln Gly Leu Ile Ser Leu Arg Ile Leu Asp Leu Ser Arg Asn Leu
   385                 390                 395                 400
   Ile His Glu Ile His Ser Arg Ala Phe Ala Thr Leu Gly Pro Ile Thr
                   405                 410                 415
   Asn Leu Asp Val Ser Phe Asn Glu Leu Thr Ser Phe Pro Thr Glu Gly
               420                 425                 430
   Leu Asn Gly Leu Asn Gln Leu Lys Leu Val Gly Asn Phe Lys Leu Lys
           435                 440                 445
   Glu Ala Leu Ala Ala Lys Asp Phe Val Asn Leu Arg Ser Leu Ser Val
       450                 455                 460
   Pro Tyr Ala Tyr Gln Cys Cys Ala Phe Trp Gly Cys Asp Ser Tyr Ala
   465                 470                 475                 480
   Asn Leu Asn Thr Glu Asp Asn Ser Leu Gln Asp His Ser Val Ala Gln
                   485                 490                 495
   Glu Lys Gly Lys Cys Met Ser Thr Ala Asp Ala Ala Asn Val Thr Ser
               500                 505                 510
   Thr Leu Glu Asn Glu Glu His Ser Gln Ile Ile Ile His Cys Thr Pro
           515                 520                 525
   Ser Thr Gly Ala Phe Lys Pro Cys Glu Tyr Leu Leu Gly Ser Trp Met
       530                 535                 540
   Ile Arg Leu Thr Val Trp Phe Ile Phe Leu Val Ala Leu Phe Phe Asn
   545                 550                 555                 560
   Leu Leu Val Ile Leu Thr Thr Phe Ala Ser Cys Thr Ser Leu Pro Ser
                   565                 570                 575
   Ser Lys Leu Phe Ile Gly Leu Ile Ser Val Ser Asn Leu Phe Met Gly
               580                 585                 590
   Ile Tyr Thr Gly Ile Leu Thr Phe Leu Asp Ala Val Ser Trp Gly Arg
           595                 600                 605
   Phe Ala Glu Phe Gly Ile Trp Trp Glu Thr Gly Ser Gly Cys Lys Val
       610                 615                 620
   Ala Gly Phe Leu Ala Val Phe Ser Ser Glu Ser Ala Ile Phe Leu Leu
   625                 630                 635                 640
   Met Leu Ala Thr Val Glu Arg Ser Leu Ser Ala Lys Asp Ile Met Lys
   645 650 655
   Asn Gly Lys Ser Asn His Leu Lys Gln Phe Arg Val Ala Ala Leu Leu
   660 665 670
   Ala Phe Leu Gly Ala Thr Val Ala Gly Cys Phe Pro Leu Phe His Arg
   675 680 685
   Gly Glu Tyr Ser Ala Ser Pro Leu Cys Leu Pro Phe Pro Thr Gly Glu
   690 695 700
   Thr Pro Ser Leu Gly Phe Thr Val Thr Leu Val Leu Leu Asn Ser Leu
   705 710 715 720
   Ala Phe Leu Leu Met Ala Val Ile Tyr Thr Lys Leu Tyr Cys Asn Leu
   725 730 735
   Glu Lys Glu Asp Leu Ser Glu Asn Ser Gln Ser Ser Met Ile Lys His
   740 745 750
   Val Ala Trp Leu Ile Phe Thr Asn Cys Ile Phe Phe Cys Pro Val Ala
   755 760 765
   Phe Phe Ser Phe Ala Pro Leu Ile Thr Ala Ile Ser Ile Ser Pro Glu
   770 775 780
   Ile Met Lys Ser Val Thr Leu Ile Phe Phe Pro Leu Pro Ala Cys Leu
   785 790 795 800
   Asn Pro Val Leu Tyr Val Phe Phe Asn Pro Lys Phe Lys Glu Asp Trp
   805 810 815
   Lys Leu Leu Lys Arg Arg Val Thr Lys Lys Ser Gly Ser Val Ser Val
   820 825 830
   Ser Ile Ser Ser Gln Gly Gly Cys Leu Glu Gln Asp Phe Tyr Tyr Asp
   835 840 845
   Cys Gly Met Tyr Ser His Leu Gln Gly Asn Leu Thr Val Cys Asp Cys
   850 855 860
   Cys Glu Ser Phe Leu Leu Thr Lys Pro Val Ser Cys Lys His Leu Ile
   865 870 875 880
   Lys Ser His Ser Cys Pro Ala Leu Ala Val Ala Ser Cys Gln Arg Pro
   885 890 895
   Glu Gly Tyr Trp Ser Asp Cys Gly Thr Gln Ser Ala His Ser Asp Tyr
   900 905 910
   Ala Asp Glu Glu Asp Ser Phe Val Ser Asp Ser Ser Asp Gln Val Gln
   915 920 925
   Ala Cys Gly Arg Ala Cys Phe Tyr Gln Ser Arg Gly Phe Pro Leu Val
   930 935 940
   Arg Tyr Ala Tyr Asn Leu Pro Arg Val Lys Asp
   945 950 955